(19) Europäisches Patentamt
European Patent Office
Office européen des brevets

(11) EP 4 389 909 A1

(12) **EUROPEAN PATENT APPLICATION**

(43) Date of publication:
**26.06.2024 Bulletin 2024/26**

(21) Application number: **23218578.5**

(22) Date of filing: **20.12.2023**

(51) International Patent Classification (IPC):
***C12Q 1/37*** *(2006.01)* ***G01N 33/573*** *(2006.01)*
***G01N 33/58*** *(2006.01)* ***G01N 33/68*** *(2006.01)*

(52) Cooperative Patent Classification (CPC):
**G01N 33/6896; C12Q 1/37; G01N 33/573; G01N 33/582;** G01N 2333/90

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.12.2022 GB 202219239**

(71) Applicant: **Esya Limited**
**London W12 0BZ (GB)**

(72) Inventor: **MODI, Souvik**
**London, W12 0BZ (GB)**

(74) Representative: **Sagittarius IP**
**Marlow International**
**Parkway**
**Marlow SL7 1YL (GB)**

Remarks:
The complete document including Reference Table(s) and the Sequence Listing(s) can be downloaded from the EPO website

(54) **NOVEL SENSOR**

(57) The invention relates to nucleic acid-based sensors and their use in determining enzyme activity, disease detection, and monitoring the effectiveness of enzyme inhibitors.

EP 4 389 909 A1

## Description

## FIELD OF THE INVENTION

The invention relates to nucleic acid-based sensors and their use in determining enzyme activity, disease detection, and monitoring the effectiveness of enzyme inhibitors.

## BACKGROUND OF THE INVENTION

Alzheimer's disease (AD) is a neurodegenerative disease that results in progressive deterioration of cognitive function (Dunn et al., 2021). AD neuropathology is defined by the presence of amyloid-β (Aβ) deposits that form extracellular plaques and intracellular neurofibrillary tangles, composed of hyperphosphorylated tau (Serrano-Pozo et al, 2021). Presence of Aβ has been posited to be the driving force behind AD pathogenesis, with tau pathology suggested as a downstream consequence of Aβ plaque formation (Götz et al., 2001; He et al., 2018). Aβ plaque formation is initiated by the amyloidogenic pathway, through which Amyloid Precursor Protein (APP) is cleaved by the beta-site APP cleaving enzyme 1 (BACE1) otherwise referred to as beta secretase (β-secretase) (Hampel et al, 2021; Cai et al., 2001). BACE1 mediated APP cleavage leads to secretion of a β-APP fragment (APPsβ) and a membrane bound C-terminal C99 fragment (C99) (Huse et al., 2002). C99 is subsequently cleaved by gamma secretase (γ-secretase) resulting in formation and secretion of Aβ, which owing to its insoluble nature is prone to aggregation and forms the main component of senile extracellular plaques in AD (Bartzokis et el., 2007). BACE1 activity is therefore posited to play a central role in the pathogenesis of AD.

APP cleavage by BACE1 has been shown to occur not at the plasma membrane, but rather, within early endosomal compartments (Sannerud et al., 2011). So far, the majority of methods for assessing BACE1 activity and inhibition have been restricted to *in vitro* studies that involve biochemical assessment using total cell lysate. These approaches fail to fully illustrate the activity of BACE1 in AD pathogenesis due to an inability to demonstrate enzymatic activity in viable cells. In addition, these approaches do not permit accurate assessment of BACE1 activity in response to clinical inhibitors inside the cellular compartments most implicated in production of Aβ.

There is therefore a need to develop novel sensors to monitor BACE1 activity in cells and organelles to support the study of Aβ pathogenesis *in vivo* as well as to help develop novel drugs that inhibit elevated BACE1 activity in AD. Sensors that be used to monitor activity of other enzymes in cells and organelles may be similarly useful.

## SUMMARY OF THE INVENTION

According to a first aspect of the invention, there is provided a nucleic acid-based sensor comprising:

(i) a sensing strand comprising a nucleic acid-peptide conjugate, a donor fluorophore and a quencher, wherein the peptide comprises a cleavable peptide sequence, and the donor fluorophore and quencher are conjugated to the nucleic acid-peptide conjugate such that cleavage of said peptide results in spatial separation of the donor fluorophore and quencher and an increase in fluorescence from the donor fluorophore; and
(ii) a reference strand comprising a nucleic acid sequence complementary to the nucleic acid sequence of the sensing strand and a reference fluorophore.

According to a further aspect of the invention, there is provided a method of determining the activity of an enzyme in a cell comprising:

(i) providing the nucleic acid-based sensor as defined herein to the cell; and
(ii) measuring the fluorescence intensity of the donor fluorophore.

According to a further aspect of the invention, there is provided a method of detecting, diagnosing, or monitoring a disease or disorder in a subject comprising:

(i) determining the activity of an enzyme in a cell obtained from said subject using the methods described herein; and
(ii) comparing the activity measured in (i) to a control or reference level of activity to detect, diagnose or monitor the disease or disorder.

According to a further aspect of the invention, there is provided a method of determining the effectiveness of an inhibitor of a target enzyme comprising:

(i) providing the enzyme inhibitor to the cell;
(ii) determining the activity of the target enzyme in a cell using the method as defined herein; and
(iii) comparing the activity measured in said cell with a control, such that a reduction in activity compared to the control is indicative of the effectiveness of the inhibitor of the target enzyme.

## BRIEF DESCRIPTION OF THE FIGURES

**Figure 1: Representative embodiment of the nucleic acid-based sensor.** Circle = Donor Fluorophore; Triangle = Reference Fluorophore; Star = Quencher.

**Figure 2: Working principle of the nucleic acid-based sensor.** Circle = Donor Fluorophore; Star = Quencher. The donor fluorophore and quencher are conjugated to the nucleic acid-peptide conjugate such that when the peptide strand is intact (i.e., not cleaved), the quencher decreases the fluorescence from the donor fluorophore. Cleavage of said peptide results in spatial separation of the donor fluorophore and quencher. The quencher is therefore no longer able to quench the fluorescence of the donor fluorophore, and there is an increase in fluorescence from said donor fluorophore.

**Figure 3: Designing sensors for an *in vitro* BACE1 activity assay.** Circle = Green Donor Fluorophore EDANS; Triangle = Red Reference Fluorophore Alexa Fluor 647; Star = Orange Quencher DABCYL. The developed sensor comprises two complementary strands (the sensing strand, SecVn, and the reference strand, ES3). The design is divided into three independent sensors by varying the SecVn strand. To generate SecV, SecOff, and SecOn sensors, $\beta$-sec$^{V1}$/$\beta$-sec$^{V2}$, $\beta$-SecOFF$^{V1}$/$\beta$-SecOFF$^{V2}$ or $\beta$-SecON$^{V1}$/$\beta$-secON$^{V2}$ strands were employed as the SecVn strand respectively. **(A)** In the SecOff sensor, the FRET pair is spaced with a non-cleavable HEG linker, resulting in a constitutively 'off' state sensor. **(B)** The SecV sensor (an embodiment of the present invention) has a BACE1 substrate carrying a FRET pair. In the absence of BACE1, DABCYL quenches EDANS fluorescence, leading to a lower Green/Red (G/R) ratio. BACE1 cleaves the target sequence at Asp(D)+1 BACE-1 cleavage site (dashed line), releasing the DABCYL quencher. This relieves its quenching effect on EDANS fluorescence leading to higher G/R ratios. (C) The SecOn sensor is a constitutively 'ON' sensor that lacks the quencher and has a DNA duplex carrying EDANS and reference fluorophore without BACE1 substrate.

**Figure 4: *In vitro* activity of sensors. (A)** *In vitro* BACE1 activity assay showing sensor performance. Fluorescence from SecOff, SecV and SecOn was measured in the presence and absence of BACE1. Ratio of donor (G) and reference (R) fluorophore plotted over different conditions. Whisker-Box plot with mean and standard error of the mean (S.E.M) is shown. Student's t-test with Welch's correction, * $p<0.05$. **(B)** Specificity of BACE1 sensor *in vitro* in the presence of different secretase inhibitors. Whisker-Box plot with mean and S.E.M is shown. One-way ANOVA with Bonferroni post-hoc test, *** $p<0.001$ (C) Sensitivity of BACE1 sensor in the presence of varying amount of BACE1 enzyme. 1.5 $\mu$M SecV sensor was incubated with 0.4 ng to 640 ng purified BACE1 enzyme and fluorescence was monitored over time.

**Figure 5: Localization of BACE1 sensor in the early endosome.** Single plane widefield images showing colocalization of SecV and early endosomal marker Rab5 RFP **(A)**, $SecOn_{647}$ and late endosomal marker Rab7 GFP **(B)** and SecV and lysosomal marker Lamp1 RFP **(C).** Cells were labelled for 30 min with SecV or $SecOn_{647}$ sensor and imaged either immediately (A) for early endosomes or chased for 180 min (B) and 360 min (C) for trafficking into late endosome and lysosomes respectively. Expanded view of endosomes marked with insert box. Scale bar, 5 $\mu$m .

**Figure 6: Quantification of colocalization. (A)** Quantification of colocalization of SecV with early endosomes after pulse or after 180 min of chase. Student's t-test with Welch's correction. **(B)** Quantification of colocalization of BACE1 sensor with late endosomes and lysosomes after pulse or after 180 min and 360 min of chase respectively. Whisker-Box plot with mean and S.E.M is shown.

**Figure 7: Spatio-temporal measurement of BACE1 activity inside cells. (A)** Temporal BACE1 activity map of endosomal organelles using SecV sensor. Top row: Pseudocolour G/R map of cells typically marked with SecV after different chase period. Bottom row: Corresponding grayscale images. Expanded view of endosomes marked with insert box. Scale bar, 5 $\mu$m. **(B)** Quantification of images shown in (A). BACE1 activity was represented as mean G/R ratio from different endosomal organelles and plotted. Each dots represent one cell. Only endosomes

that colocalized with Rab5 GFP is quantified for 30 min pulse while for 180 min chase, endosomes that do not colocalizes with Rab5 GFP were quantified. Whisker-Box plot with mean and S.E.M is shown. One- way ANOVA followed by Bonferroni's post hoc test, *p*<0.01.

**Figure 8: Specificity of the SecV sensor inside late endosomes. (A)** Pseudocolour G/R map of cells in the presence and absence of secretase inhibitors. Cells were labeled with SecV and imaged after 180 min chase at 37 °C. DMSO is used as no inhibitor control. Expanded view of endosomes marked with white box. Scale bar, 5 μm. **(B)** Quantification of BACE1 activity as fold change in the presence of β-secretase and γ-secretase inhibitors. Each dot represented as an independent experiment. Permutation t-test, p<0.05.

**Figure 9: Monitoring BACE1 activity in cells from Alzheimer's disease patients.** Pseudocolour G/R map of BACE1 activity from healthy, familial AD or sporadic AD patients. Cells were labelled for 60 min with either SecOff (first panel) or SecV sensor and imaged after 180 min chase at 37 °C. Expanded view of endosomes marked with insert box. Scale bar, 5 μm.

**Figure 10: Quantification of BACE1 activity in cells from Alzheimer's disease patients.** Quantification of images shown in Figure 9. More than 100 endosomes over 10 cells quantified. Each dot represented one individual (either healthy or with Alzheimer's disease). Fold change of activity quantified after normalizing to SecOff and plotted as groups of Healthy and AD **(A)**, Healthy and AD with different mutations **(B)** and Healthy and individuals with 50 % risk of developing AD **(C).** Permutation *t*-test, *p*<0.05.

**Figure 11: Generation of SecV, SecOff, SecOn and $SecOn_{647}$ sensors.** To generate the SecV, SecOff, SecOn and $SecOn_{647}$ sensors, β-$sec^{V1}$/β-$sec^{V2}$, β-$SecOFF^{V1}$/β-$SecOFF^{V2}$, β-$SecON^{V1}$/β-$secON^{V2}$ or ES4 strands respectively were annealed to ES3.

## DETAILED DESCRIPTION OF THE INVENTION

### *Nucleic Acid-based Sensor*

The present inventors have developed a novel nucleic acid-based sensor which allows for quantitative measurement of enzyme activity within cells. A representative embodiment is shown in **Figure 1.**

Therefore, according to a first aspect of the invention, there is provided a nucleic acid-based sensor comprising:

(i) a sensing strand comprising a nucleic acid-peptide conjugate, a donor fluorophore and a quencher, wherein the peptide comprises a cleavable peptide sequence, and the donor fluorophore and quencher are conjugated to the nucleic acid-peptide conjugate such that cleavage of said peptide results in spatial separation of the donor fluorophore and quencher and an increase in fluorescence from the donor fluorophore; and

(ii) a reference strand comprising a nucleic acid sequence complementary to the nucleic acid sequence of the sensing strand and a reference fluorophore.

### *Nucleic Acid*

In one embodiment, the nucleic acid is selected from the group consisting of: DNA, RNA or a nucleic acid analogue such as a peptide nucleic acid (PNA), or any combination thereof. Preferably, the nucleic acid is DNA.

The nucleic acids of the disclosure may be 20-120 nucleic acids in length.

In one embodiment, the nucleic acid of the sensor is a ligand for one or more scavenger receptors (SRs).

Exogenous DNA is known to cause immune stimulation. The innate immune system utilizes a number of pattern recognition receptors (PRRs) to recognize non-self microbial products via pathogen-associated molecular patterns (PAMPs). An example of PRRs are the Toll-like receptors (TLRs) which are expressed on the cell surface and in endosomal compartments. In one embodiment, the nucleic acid does not comprise any sequences that stimulate a TLR pathway. For example, it is known that unmethylated CpG DNA motifs have the ability to stimulate immune responses through an endosomal TLR9 pathway. Therefore, in one embodiment, the nucleic acid does not comprise any CpG motifs. Suitably the nucleic acid does not form tertiary structures other than duplexes, e.g., triplexes or quadruplexes (e.g., G-quadruplexes).

The term "complementary" as used herein in reference to complementary sequences refers to the capacity for two sequences to hybridise through nucleotide base pairs consisting of two complementary nucleotides bound to each other by hydrogen bonds. In canonical Watson-Crick base pairing in DNA, adenine (A) forms a base pair with thymine (T), and guanine (G) forms a base pair with cytosine (C). In canonical Watson-Crick base pairing in RNA, thymine is

replaced by uracil (U). Base pairs may also be formed through non-canonical base pairing, having hydrogen bonding patterns which differ from the patterns observed in Watson-Crick base pairs, or through unnatural base pairs. References to "complementary" as used herein also include wherein the nucleic acid sequences of the reference strand and sensing strand are partially complementary such that the strands are capable of forming sufficient base pairs to produce a duplex. Suitably the degree of complementarity (i.e., the percentage of complementary nucleotides) is at least 80% e.g., at least 85% e.g., at least 90% e.g., at least 95%, 96%, 97%, 98% or 99%. In one embodiment the degree of complementarity is 100%.

*Donor Fluorophores and Quenchers*

In the present invention, the sensing strand allows for the detection of enzymatic activity by utilizing the process of fluorescence quenching, wherein the fluorescence intensity of a fluorophore is reduced through an interaction with another molecule. The term "donor fluorophore" as used herein refers to a fluorophore which can absorb energy (e.g., by excitation radiation) and transfer or "donate" that energy to another molecule. The term "quencher" as used herein refers to a molecule which, when in close proximity to a suitable donor fluorophore, is capable of decreasing or "quenching" the fluorescence of said donor. In the present invention, the donor fluorophore and quencher are conjugated to the nucleic acid-peptide conjugate such that when the peptide strand is intact (i.e., not cleaved), the quencher decreases the fluorescence from the donor fluorophore. Cleavage of said peptide results in spatial separation of the donor fluorophore and quencher. The quencher is therefore no longer able to quench the fluorescence of the donor fluorophore, and there is an increase in fluorescence from said donor fluorophore (see Figure 2).

Quenching may occur by any of several mechanisms including Fluorescence Resonance Energy Transfer (FRET, also known as Förster resonance energy transfer) and Dexter Electron Transfer. In one embodiment, quenching occurs by FRET, wherein upon excitation the donor fluorophore is capable of transferring energy to the quencher without emission of a photon. Typically, the quencher is a fluorophore, and the absorbed energy is re-emitted as light, however the quencher may also be a dark quencher, which dissipates the absorbed energy as heat. Preferably, the quencher is a fluorophore.

One requirement of FRET is that there is an overlap between the donor fluorophore's emission spectrum and the quencher's absorbance spectrum. Examples of suitable FRET donor-quencher pairs which fulfil this requirement are known in the art and may include, without limitation: EDANS and DABCYL, HiLyte Fluor 488 and QXL520, IAEDANS and Fluorescein, Alexa Fluor 488 and Alexa Fluor 555, Alexa Fluor 594 and Alexa Fluor 647, or mOrange and mCherry. In one embodiment, the donor and quencher fluorophores are respectively EDANS and DABCYL or HiLyte Fluor 488 and QXL520.

**Excitation and Emission Peaks of Commercial Fluorophores:**

| Fluorophore | Excitation (nm) | Emission (nm) |
|---|---|---|
| HiLyte Fluor 488 | 490 | 520 |
| Alexa Fluor 488 | 490 | 525 |
| Alexa Fluor 555 | 553 | 568 |
| Alexa Fluor 594 | 590 | 617 |
| Alexa Fluor 647 | 650 | 671 |

As discussed above, quenching relies on the quencher being in close proximity to a suitable donor fluorophore. The maximum distance between the quencher and donor fluorophore for quenching to occur will vary depending on the quenching mechanism. For example, the range over which FRET can occur is limited to about 10 nm or 100 Å. Therefore, in one embodiment the donor fluorophore and quencher are each conjugated to the nucleic acid-peptide conjugate such that the distance between the donor fluorophore and quencher is less than 10nm.

Further, in the present invention cleavage of the peptide portion of the nucleic acid-peptide conjugate results in spatial separation of the donor fluorophore and quencher. Therefore, it would be clear to the skilled person that the donor fluorophore and quencher should be conjugated to the nucleic acid-peptide conjugate, such that the donor fluorophore and quencher are positioned on opposite sides of the cleavage site. In one embodiment, both the donor fluorophore and quencher are conjugated to the peptide portion of the sensing strand, such as wherein the donor fluorophore and quencher are conjugated to the N-terminus and C-terminus amino acids of the peptide portion respectively, or vice versa.

Alternatively, the donor fluorophore may be conjugated to the nucleic acid portion of the sensing strand, while the quencher is conjugated to the peptide portion of the sensing strand, or vice versa.

*Reference Fluorophore*

The reference strand comprises a reference fluorophore which is conjugated to the reference strand. The term "reference fluorophore" as used herein refers to a fluorophore wherein the fluorescence intensity of the fluorophore does not change upon cleavage of the cleavable peptide sequence. In one embodiment, the donor fluorophore and reference fluorophore have different emission wavelengths.

The skilled person would be able to identify a suitable fluorophore from the fluorophores known in the art, which include, without limitation: Alexa Fluor 350, Alexa Fluor 405, Alexa Fluor 488, Alexa Fluor 532, Alexa Fluor 546, Alexa Fluor 555, Alexa Fluor 561, Alexa Fluor 568, Alexa Fluor 594, Alexa Fluor 647, Alexa Fluor 660, Alexa Fluor 680, Alexa Fluor 700, Alexa Fluor 750, Atto 390, Atto 425, Atto 465, Atto 488, Atto 495, Atto 520, Atto 532, Atto 540Q, Atto 550, Atto 565, Atto 580Q, Atto 590, Atto 594, Atto 610, Atto 611X, Atto 612Q, Atto 620, Atto 633, Atto 647, Atto 647N, Atto 655, Atto 680, Atto 700, Atto 725, Atto 740, BODIPY FL, Coumarin, Cy3, Cy5, DyLight 350, DyLight 405, DyLight 488, DyLight 550, DyLight 594, DyLight 633, DyLight 650, DyLight 680, DyLight 755, DyLight 800, Fluorescein (FITC), Oregon Green, Pacific Blue, Pacific Green, Pacific Orange, PE-Cyanine7, PerCP-Cyanine5.5, Tetramethylrhodamine (TRITC), and Texas Red. In one embodiment, the reference fluorophore is Alexa Fluor 647.

Critically, the known stoichiometry of nucleic acid hybridization allows the addition of the reference fluorophore at a known ratio to the donor fluorophore. This results in a ratiometric sensor, which is useful for correcting for potential cell to cell or inter-assay variation. In one embodiment, the donor fluorophore and reference fluorophore are in a 1:1 ratio.

The donor fluorophore, reference fluorophore and quencher of the present invention can be conjugated to the strands of the sensor directly or indirectly by a variety of techniques known in the art. For example, the fluorophores may include derivatives that have been modified to facilitate conjugation to another reactive molecule, such as amine-reactive derivatives (e.g., isothiocyanate derivatives, succinimidyl ester derivatives) and/or, thiol-reactive derivates (e.g. maleimide derivatives). The fluorophores may also be click-reactive for incorporation by click chemistry. For example, the fluorophore may comprise (or may be derivatized to comprise) an azide or alkyne moiety.

The fluorophores may be conjugated to the peptide portion via functional or reactive groups on the peptide. These are typically formed from the side chains of particular amino acids found in the polypeptide polymer. Such reactive groups may be a cysteine side chain, a lysine side chain, a cysteamine residue, or an N-terminal amine group or any other suitable reactive group. Examples of reactive groups of natural amino acids are the thiol group of cysteine, the amino group of lysine, the carboxyl group of aspartate or glutamate, the guanidinium group of arginine, the phenolic group of tyrosine or the hydroxyl group of serine. Non-natural amino acids can provide a wide range of reactive groups including an azide, a keto-carbonyl, an alkyne, a vinyl, or an aryl halide group. The amino and carboxyl group of the termini of the polypeptide can also serve as reactive groups.

Similarly, the fluorophores can be conjugated to the nucleic acid molecules by a variety of techniques. The fluorophore can be conjugated at the 5’ or 3’ end of the nucleic acid, located internally in the nucleotide sequence of the nucleic acid, or attached to spacer arms extending from the nucleic acid. Nucleic acid molecules may also incorporate functionalizing reagents having one or more sulfhydryl, amino or hydroxyl moieties into the nucleic acid sequence.

One benefit of the use of fluorophores in the present sensor is that most commercially available fluorophores are insensitive to pH (including, for example, Alexa Fluor 350, Alexa Fluor 405, Alexa Fluor 488, Alexa Fluor 532, Alexa Fluor 546, Alexa Fluor 555, Alexa Fluor 561, Alexa Fluor 568, Alexa Fluor 594, Alexa Fluor 647, Alexa Fluor 660, Alexa Fluor 680, Alexa Fluor 700, Alexa Fluor 750, Atto 390, Atto 425, Atto 465, Atto 488, Atto 495, Atto 520, Atto 532, Atto 540Q, Atto 550, Atto 565, Atto 580Q, Atto 590, Atto 594, Atto 610, Atto 611X, Atto 612Q, Atto 620, Atto 633, Atto 647, Atto 647N, Atto 655, Atto 680, Atto 700, Atto 725, Atto 740, BODIPY FL, Coumarin, Cy5, DyLight 350, DyLight 405, DyLight 488, DyLight 550, DyLight 594, DyLight 633, DyLight 650, DyLight 680, DyLight 755, DyLight 800, Pacific Blue, Pacific Green, Pacific Orange, PE-Cyanine7, PerCP-Cyanine5.5, Tetramethylrhodamine (TRITC), and Texas Red). Therefore, the sensor may be employed in acidic environments such as within acid organelles (e.g., endosomes and lysosomes). Therefore, in one embodiment, the fluorophores employed in the sensor are pH insensitive.

*Peptide Sequence*

The peptide sequence of the disclosure may be 8-12 amino acids in length.

In one embodiment, the cleavable sequence is a sequence that is susceptible to cleavage by an enzyme, such as a protease. Cleavage occurs at a cleavage site, typically a site between two particular residues according to the specificity of the enzyme. Examples of enzymes capable of cleavage include, but are not limited to: cathepsin, proteases of the matrix metalloprotease family, and proteases of the secretase family (e.g., $\alpha$-secretase, $\beta$-secretase and $\gamma$-secretase). In one embodiment, the enzyme is a secretase. In a further embodiment the secretase is beta-site APP cleaving

enzyme 1 (BACE1).

One application envisioned for the sensor of the present invention is for monitoring beta-site APP cleaving enzyme 1 (BACE1) activity. Therefore, in one embodiment the cleavable peptide sequence is a BACE1 sensitive peptide sequence. The term "BACE1 sensitive peptide sequence" as used herein refers to a peptide sequence that can be specifically recognized and cleaved by BACE1. In a further embodiment, the cleavable peptide sequence sensitivity is selective for BACE1, such that the peptide sequence can only be cleaved by BACE1.

BACE1 is known to cleave APP at two sites: the major Glu11 site of the amyloid β (Aβ) domain, yielding the nonamyloidogenic C89 and resulting in truncated Aβ production; and the minor Asp1 site, generating C99 and leading to Aβ generation. Shifting the preferential cleavage site from Glu11 to Asp1 results in higher Aβ production and facilitates Aβ plaque formation under pathological conditions (Zhang et al, 2017). The 'Swedish' APP mutation, which differs to the native APP sequence by a KM670/671NL substitution in the APP770 isoform (or the corresponding position in another APP isoform, e.g., K595N/M596L in the APP695 isoform), strongly shifts the BACE1 cleavage site from Glu11 to Asp1, resulting in a higher C99/C89 ratio and promoting Aβ generation (Deng et al., 2013). Therefore, in one embodiment, the cleavable peptide sequence is a sequence derived from the 'Swedish' APP mutation. In a further embodiment, the cleavable peptide sequence is EVNLDAEF (SEQ ID NO: 1).

*Targeting Moieties*

In one embodiment, the sensor further comprises a targeting moiety. The term "targeting moiety" as used herein refers to a molecule such as a nucleic acid, small molecule, or polypeptide that has an affinity for a certain target or, by virtue of its chemical makeup, is targeted to a particular location in the cell. The targeting moiety can act to target the sensor of the present invention to different subcellular locations.

In one embodiment, the targeting moiety is a nucleic acid sequence. In a further embodiment, the targeting moiety may be encoded within the DNA sequence of the sensing strand and/or the reference strand. In an alternative embodiment, the targeting moiety is a nucleic acid sequence which is directly or indirectly conjugated to the sensing strand and/or the reference strand.

In an alternative embodiment, the targeting moiety comprises a peptide directly or indirectly conjugated to the sensing strand and/or the reference strand. In a further embodiment, the targeting moiety peptide comprises one or more of a fusogenic peptide, a membrane-permeabilizing peptide, a sub-cellular localization sequence, or a cell-receptor ligand.

***Method of Determining Enzyme Activity***

According to a further aspect of the invention, there is provided a method of determining the activity of an enzyme in a cell comprising:

(i) providing the nucleic acid-based sensor as defined herein to the cell; and
(ii) measuring the fluorescence intensity of the donor fluorophore.

As previously discussed, the known stoichiometry of the hybridization of the sensing strand and reference strand allows the addition of the reference fluorophore at a known ratio to the donor fluorophore. This results in a ratiometric sensor, which is useful for correcting for potential cell to cell or inter-assay variation. Therefore, in one embodiment the method further comprises measuring the fluorescence intensity of the reference fluorophore (R) and determining a normalised value for the fluorescence intensity of the donor fluorophore (D), wherein the normalised value is the ratio D/R.

In one embodiment, the enzyme activity is measured inside an organelle. Examples of organelles include, but are not limited to acrosomes, autophagosomes, the cell membrane or cell wall, centrioles, cilium, chloroplasts, the endoplasmic reticulum, early and late endosomes, flagellum, Golgi apparatus, lysosomes, melanosomes, mitochondrion, nucleolus, nucleus, peroxisomes, proteasomes, ribosomes, vacuoles, and vesicles.

In one embodiment, the organelle is an endocytic organelle such as the endoplasmic reticulum, the Golgi apparatus, peroxisomes, lysosomes, or early and late endosomes. In one embodiment, the organelle is an acidic organelle such as a lysosome or an endosome.

In one embodiment, the cell is a somatic cell. References herein to "somatic cell" refer to any type of cell that makes up the body of an organism, excluding germ cells and undifferentiated stem cells. Somatic cells may therefore include, for example, skin, heart, muscle, nerve, bone or blood cells. In one embodiment, the somatic cell is a nerve cell, such as a cell from the central and/or peripheral nervous system. Thus, in one embodiment, the cell is a neuron. In a further embodiment, the cell is a sensory neuron. In an alternative embodiment, the cell is a motor neuron. In another embodiment, the cell is an interneuron. In a further embodiment, the neuron is a brain cell. In one embodiment, the cell

is a glial cell. In a further embodiment, the glial cell is a microglial cell. In an alternative embodiment, the glial cell is an astrocyte.

Somatic cells may be prepared from Induced Pluripotent Stem Cells (iPSCs). iPSCs are cells that have been reprogrammed to an embryonic stem cell-like state by being forced to express genes and factors important for maintaining the defining properties of embryonic stem cells. As pluripotent cells, iPSC theoretically have the ability to generate any somatic cell type. Human iPSCs have been differentiated into a variety of cell types, including adipocytes, cardiomyocytes, hematopoietic cells, pancreatic cells, and several neuronal cell types. Therefore, in one embodiment the cell is an iPSC derived neuron.

In one embodiment, the somatic cell is from an animal. In a further embodiment, the somatic cell is from a mammal. In a further embodiment, the mammal is a human. In an even further embodiment, the cell is from a human suffering from a disease or disorder, such as a disease or disorder associated with dysfunction of an endocytic organelle. Particularly, the cell is an iPSC-derived neuron from a subject with from Alzheimer’s disease.

One application envisioned for the sensor of the present invention is for monitoring beta-site APP cleaving enzyme 1 (BACE1) activity. Therefore, in one embodiment the enzyme is BACE1. In a further embodiment, the enzyme is BACE1 and the cleavable peptide sequence is a BACE1 sensitive peptide sequence such as EVNLDAEF (SEQ ID NO: 1).

The nucleic acid-based sensor may be provided to the cell by a number of methods. For example, the cell may be incubated with the sensor. In an embodiment the nucleic acid of the sensor activates pathways leading to trafficking of the sensor down the endolysosomal pathway. In one such embodiment, the nucleic acid of the sensor is a ligand for one or more scavenger receptors (SRs). Scavenger receptors recognise a wide range of negatively charged molecules including nucleic acids. The sensor will therefore bind SRs on the surface of the cell and be internalised via receptor-mediated endocytosis. The sensor may then be trafficked down the endolysosomal pathway. The skilled person would understand that the sensors of the invention may be targeted to other DNA-binding receptors on the cell surface which induce endocytosis of the ligand, including artificial chimeric receptors created by fusing a DNA binding protein or antibody to a plasma membrane expressing protein (e.g., as described in Modi et al, 2013 and Chakraborty et. al, 2021).

In one embodiment, the method additionally comprises comparing the fluorescence intensity of the donor fluorophore of the sensor with the fluorescence intensity of a donor fluorophore in a control sensor. For example, the control sensor may be a constitutively ’off’ sensor, wherein the cleavable sequence of the sensing strand is replaced by a non-cleavable sequence or linker (Figure 3A). As such, the donor fluorophore and quencher cannot be separated by enzymatic cleavage and the donor intensity is quenched throughout the course of the method, providing a good reference sensor to mimic the hypothetical state of the sensor when no enzyme is present. In one embodiment, the non-cleavable linker is a hexaethylene glycol (HEG) linker. Alternatively, the control sensor may be a constitutively ’on’ sensor that lacks the quencher (Figure 3C). A constitutively ’on’ sensor indicates the maximum signal obtainable from the donor fluorophore, mimicking the hypothetical state where the reaction between the enzyme and its substrate reach saturation (i.e., 100 % of the quencher is cleaved from the substrate). In one embodiment, the method comprises comparison of the fluorescence intensity of the donor fluorophore of the sensor with the fluorescence intensity of a donor fluorophore in a constitutively ’off’ sensor and a constitutively ’on’ sensor.

### *Disease Detection Methods*

According to a further aspect of the invention, there is provided a method of detecting, diagnosing, or monitoring a disease or disorder in a subject comprising:

(i) determining the activity of an enzyme in a cell obtained from said subject using the methods described herein; and
(ii) comparing the activity measured in (i) to a control or reference level of activity to detect, diagnose or monitor the disease or disorder.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, i.e., for drug screening and drug development.

The terms "control" or "reference level" as used herein in relation to diagnosis methods refer to a level of enzyme activity against which a test sample is compared. It will be clear to those skilled in the art that the control or reference level may be selected on a variety of basis. For example, the control or reference level may be one of that associated with subjects known to be free of the disease or may be subjects with a different disease (for example, for the investigation of differential diagnosis). The "control" may comprise a level from a healthy subject, a non-diseased subject or a level

determined for an individual earlier in the course of diagnosis. Regarding the latter, the reference level is useful in disease monitoring to determine changes in biomarker levels which are indicative of disease progression or regression. Comparison with a control is well known in the field of diagnostics.

It will be understood that it is not necessary to measure healthy/non-diseased controls for comparative purposes on every occasion because once the 'normal range' is established it can be used as a benchmark for all subsequent tests. A normal range can be established by obtaining samples from multiple control subjects without the disease. Results (i.e., enzyme activity levels) for subjects suspected to have the disease can then be examined to see if they fall within or outside of the respective normal range. Use of a 'normal range' is standard practice for the detection of disease.

References to "subject" or "patient" are used interchangeably herein. In one embodiment, the patient is a human patient. In one embodiment, the patient is a (non-human) animal. The use, panels and methods described herein are preferably performed *in vitro*. References to acts carried out on a sample or cell "obtained" from a subject are intended to encompass acts carried out on a body fluid sample already obtained or "obtainable" from a subject.

In one embodiment, the disease or disorder associated with dysfunction of an endocytic organelle such as lysosomes, endosomes, or the Golgi apparatus. In one embodiment, the disease or disorder associated with lysosome dysfunction such as lysosomal storage disease. Examples of lysosomal storage disease include, but are not limited to: Gaucher disease, Niemann-Pick disease, Fabry disease, Tay-Sachs disease, Mucopolysaccharidoses diseases and Pompe disease. Increasing evidence suggest that dysfunction of lysosomes also plays a key role in the development of Alzheimer's disease (AD) and other neurodegenerative diseases associated with protein misprocessing and accumulation. Therefore, in one embodiment, the disease or disorder is a neurodegenerative disease. In a further embodiment, the disease or disorder is a neurodegenerative disease associated with increased accumulation of β-amyloid, such as Alzheimer's disease.

BACE1 is known to cleave at the minor Asp1 site, generating C99 and leading to Aβ generation. The present inventors have demonstrated that BACE1 activity inside the endosomal system, when measured with the sensor of the present invention, is increased in Alzheimer's disease patients compared to a healthy subject (Figure 10A). Therefore, in one embodiment, the disease or disorder is Alzheimer's disease, and the enzyme is BACE1. In a further embodiment, the cleavable peptide sequence is a BACE1 sensitive peptide sequence such as EVNLDAEF (SEQ ID NO: 1). In an even further embodiment, the control or reference level is the level from a healthy subject, such that an increase in BACE1 activity compared to the control or reference level is indicative of Alzheimer's disease.

The present inventors have also demonstrated that BACE1 activity is increased in AD patients with PSEN-1 mutations but remains unaltered in AD patients with APOE4 alleles (Figure 10B). Therefore, in one embodiment, the disease or disorder is Alzheimer's disease in patients carrying the PSEN-1 mutation.

Further, the present inventors have demonstrated that the sensor of the present invention can detect increased BACE1 activity in pre-symptomatic AD carriers (Patients with 50% risk of developing AD) (Figure 10C). Therefore, in one embodiment, the disease or disorder is pre-symptomatic Alzheimer's disease.

***Methods of Monitoring Drug Effectiveness***

It is also envisaged that the sensor of the present invention may be useful in developing novel drugs that inhibit enzyme activity. Therefore, according to a further aspect of the invention, there is provided a method of determining the effectiveness of an inhibitor of a target enzyme comprising:

(i) providing the enzyme inhibitor to the cell;
(ii) determining the activity of the target enzyme in a cell using the method as defined herein; and
(iii) comparing the activity measured in said cell with a control, such that a reduction in activity compared to the control is indicative of the effectiveness of the inhibitor of the target enzyme.

The term "control" as used herein in relation to methods of determining the effectiveness of an inhibitor diagnosis methods refer to a level of enzyme activity against which a test sample is compared. It will be clear to those skilled in the art that the control level may be selected on a variety of basis. For example, the control or reference level may be a cell which has not been provided with the inhibitor or may be the enzyme level measured in the cell before the enzyme inhibitor was provided. Comparison with a control is well known in the field of drug discovery.

**CLAUSES**

The invention is further illustrated by reference to the following clauses:

Clause 1. A nucleic acid-based sensor comprising:

(i) a sensing strand comprising a nucleic acid-peptide conjugate, a donor fluorophore and a quencher, wherein the peptide comprises a cleavable peptide sequence, and the donor fluorophore and quencher are conjugated to the nucleic acid-peptide conjugate such that cleavage of said peptide results in spatial separation of the donor fluorophore and quencher and an increase in fluorescence from the donor fluorophore; and
(ii) a reference strand comprising a nucleic acid sequence complementary to the nucleic acid sequence of the sensing strand and a reference fluorophore.

Clause 2. The nucleic acid-based sensor as defined in clause 1, wherein the nucleic acid is DNA.

Clause 3. The nucleic acid-based sensor as defined in clause 1 or clause 2, wherein the quenching occurs by Fluorescence Resonance Energy Transfer (FRET).

Clause 4. The nucleic acid-based sensor as defined in any of clauses 1 to 3, wherein the donor and quencher fluorophores are respectively EDANS and DABCYL or HiLyte Fluor 488 and QXL520.

Clause 5. The nucleic acid-based sensor as defined in clauses 1 to 4, wherein the fluorophores employed in the sensor are pH insensitive.

Clause 6. The nucleic acid-based sensor as defined in any of clauses 1 to 5, wherein the cleavable peptide sequence is a beta-site APP cleaving enzyme 1 (BACE1) sensitive peptide sequence.

Clause 7. The nucleic acid-based sensor as defined in any of clauses 1 to 6, wherein the cleavable peptide sequence sensitivity is selective for BACE1.

Clause 8. The nucleic acid-based sensor as defined in any of clauses 1 to 7, wherein the cleavable peptide sequence is EVNLDAEF (SEQ ID NO: 1).

Clause 9. The nucleic acid-based sensor as defined in any of clauses 1 to 8, wherein the reference fluorophore is Alexa Fluor 647.

Clause 10. The nucleic acid-based sensor as defined in any of clauses 1 to 9, wherein the donor fluorophore and reference fluorophore have different emission wavelengths.

Clause 11. The nucleic acid-based sensor as defined in any of clauses 1 to 10, wherein the donor fluorophore and reference fluorophore are in a 1:1 ratio.

Clause 12. A method of determining the activity of an enzyme in a cell comprising:

(i) providing the nucleic acid-based sensor as defined in any of clauses 1 to 11 to the cell; and
(ii) measuring the fluorescence intensity of the donor fluorophore.

Clause 13. The method as defined in clause 12, wherein the method further comprises measuring the fluorescence intensity of the reference fluorophore (R), and determining a normalised value for the fluorescence intensity of the donor fluorophore (D), wherein the normalised value is the ratio D/R.

Clause 14. The method as defined in clause 12 or clause 13, wherein in the activity is measured inside an organelle.

Clause 15. The method as defined in any of clauses 12 to 14, wherein in the organelle is an acidic organelle.

Clause 16. The method as defined in clause 15, wherein in the organelle is a lysosome or an endosome.

Clause 17. The method as defined in any of clauses 12 to 16, wherein in the cell is a neuron.

Clause 18. The method as defined in any of clauses 12 to 17, wherein in the enzyme is BACE1.

Clause 19. A method of detecting, diagnosing, or monitoring a disease or disorder in a subject comprising:

(i) determining the activity of an enzyme in a cell obtained from said subject using the methods described in

any of clauses 12 to 18; and
(ii) comparing the activity measured in (i) to a control or reference level of activity to detect, diagnose or monitor the disease or disorder.

Clause 20. The method as defined in clause 19, wherein the disease or disorder is selected from: a neurodegenerative disease, PTSD, heart disease, epithelial cancers, diabetes, peroxisomal disorders.

Clause 21. The method as defined in clause 20, wherein the neurodegenerative disease is a neurodegenerative disease associated with increased generation of β-amyloid.

Clause 22. The method as defined in clause 21, wherein the disease or disorder is Alzheimer's disease.

Clause 23. A method of determining the effectiveness of an inhibitor of a target enzyme comprising:

(i) providing the enzyme inhibitor to the cell;
(ii) determining the activity of the target enzyme in a cell using the method as defined in any of clauses 12 to 18; and
(iii) comparing the activity measured in said cell with a control, such that a reduction in activity compared to the control is indicative of the effectiveness of the inhibitor of the target enzyme.

## EXAMPLES

### Materials

Oligonucleotides and conjugated peptides/fluorophores were purchased from Eurogentec® and IDT and reconstituted to a final concentration of 1 mM in nuclease-free water (Sigma-Aldrich®) and used without further purification. Bacmam organelle markers were purchased from Thermofisher UK. DAPT was purchased from Abcam. All other reagents were purchased from Sigma-Aldrich or Fisher scientific UK unless otherwise specified.

### Methods

*Oligonucleotides*

Reconstituted oligonucleotides were quantified using a NanoDrop™ One UV-Vis spectrophotometer and subsequently stored at -20°C. To generate SecV, SecOff, and SecOn sensors, 50 μM of β-sec$^{V1}$/β-sec$^{V2}$, β-SecOFF$^{V1}$/β-SecOFF$^{V2}$ or β-SecON$^{V1}$/β-secON$^{V2}$ strands (as described in Table 2 and Figure 11) respectively were annealed to 50 μM of ES3. Formation of annealed products were confirmed by gel electrophoresis in an Novex™ 10 % TBE gel run at 150 v for 30 min in 1x UltraPure™ TBE buffer (100 mM Tris HCL, 90 mM Boric acid, 1 mM EDTA) using a XCell SureLock™ gel tank and Bio-Rad® PowerPac™ 300 power supply. An analytikjena® UVP™ transilluminator was used to assess success of annealing, and sensors were stored at 4°C for up to 3 weeks.

*Table 1. Oligonucleotide and peptide sequences*

| SEQ ID NO | Sequence |
|---|---|
| SEQ ID NO: 2 | 5'-ATAACACATAACACATAACAAAATATATATCCTAGAACGAC-3' |
| SEQ ID NO: 3 | 5'-TGTCGTTCTAGGATATATATTTTGTTATGTGTTATGTGTTAT-3' (5'-T-(SEQ ID NO: 4)-3') |
| SEQ ID NO: 4 | 5'-GTCGTTCTAGGATATATATTTTGTTATGTGTTATGTGTTAT-3' |
| SEQ ID NO: 5 | RKFEADLNVE |
| SEQ ID NO: 6 | KFEADLNVEK |

*Table 2. Strand Design*

| Strand | Sequence | Sensor(s) used in | Application |
|---|---|---|---|
| ES3 | **Alexa Fluor 647N**-(SEQ ID NO: 2) | SecOn, SecOff, SecV, $SecOn_{647}$ | Used as a complementary strand in all experiments |
| β-SecON$^{V1}$ | (**EDANS**)-(SEQ ID NO: 3) | SecOn | *In vitro* BACE1 activity |
| β-SecON$^{V2}$ | **HiLyte-Fluor 488**-(SEQ ID NO: 4) | SecOn | Colocalization assay inside cells |
| β-SecOFF$^{V1}$ | **Dabcyl**-HEG-(**EDANS**)-(SEQ ID NO: 3) | SecOff | *In vitro* BACE1 activity |
| β-SecOFF$^{V2}$ | **QXL520**-HEG-(**HiLyte-Fluor 488**)-(SEQ ID NO: 4) | SecOff | *In vitro* (inhibitor assay) and BACE1 activity inside cells |
| β-sec$^{V1}$ | **Dabcyl-**(SEQ ID NO: 5)-**(EDANS)***ERC*-SMCC-HEG-(SEQ ID NO: 4) | SecV | *In vitro* BACE1 activity |
| β-sec$^{V2}$ | N-**QXL520**-(SEQ ID NO: 6)-**(HiLyte-Fluor 488)** *C*-SMCC-HEG_NH-(SEQ ID NO: 4) | SecV | *In vitro* (inhibitor assay) and BACE1 activity inside cells |
| ES4 | (SEQ ID NO: 4) | $SecOn_{647}$ | Complementary to ES3, used for colocalization assay |

*In vitro BACE1* assay

BACE1 fluorometric assay kit was purchased from Abcam® and was prepared according to manufacturer's suggestions. The BACE1 assay was performed in 100 mL reaction volume constituting 50 mL 2x β-secretase reaction buffer, 5 mL of 50 μM DNA sensor (SecV, SecOff and SecOn, Final concentration 2.5 μM and 2 μL (0.8 mg) of purified BACE1 enzyme (ab271386, Abcam) for +BACE1 well or 2 μL nuclease free water for -BACE1 wells. The reaction volume was brought to 100 5 μL by adding 1x nuclease free $H_2O$ (Ambion). The reaction was incubated for 1 h at 37 °C and transferred to an 8 well imaging chamber (Ibidi GmBH). Images were acquired in an Olympus® IX83 fluorescent microscope equipped with a Photometrics® Prime™-95B sCMOS camera in the EDANS channel (378/52 excitation and 432/36), and Alexa Fluor 647 channel (635/18 excitation, 680/42 emission). Mean intensity in the EDANS (Donor, G) channel was divided by mean intensity observed at Alexa Fluor 647 (Reference fluorophore, R) channel and the G/R ratio is plotted for comparison between with and without BACE1 wells.

*In vitro BACE1 inhibition*

The BACE1 assay was performed as described earlier with minor modifications. β-secretase inhibitor IV ((BSIV) Millipore®):

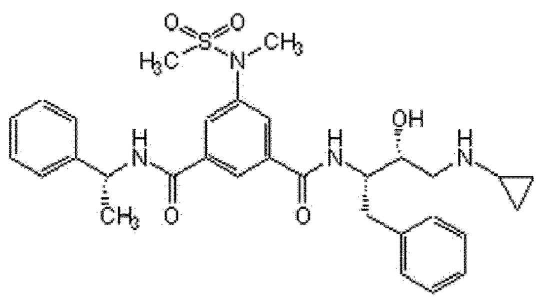

or DAPT $\gamma$-secretase inhibitor (Abcam®):

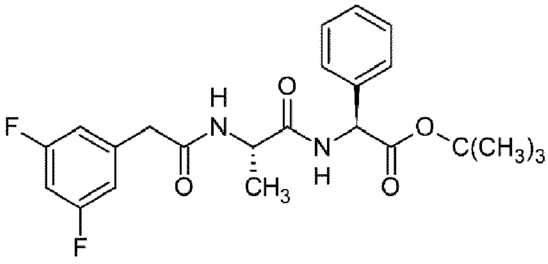

were dissolved in dimethyl sulfoxide ((DMSO) FisherScientific®). 1x $\beta$-secretase reaction buffer containing a total of 0.04 $\mu$g of recombinant human BACE1 (Abcam®) was mixed with 300 nM of BSIV or 800 nM of DAPT inhibitors and incubated for 30 min at 37 °C. 1 $\mu$M of $\beta$-secretase sensors were then added to solutions and incubated at 37 °C for a further 30 min before fluorescent quantifications were obtained using an IX83 widefield fluorescence microscope.

*Cell Culture*

Primary dermal fibroblasts from healthy individuals and AD patients were obtained from the Coriell institute and cultured at 37 °C in 5 % $CO_2$ in proliferation medium comprising high glucose DMEM (25 mM glucose, 1 mM pyruvate, 4 mM Phenol Red (Gibco™) supplemented with 10 % FBS (Gibco™), 1x NEAA (Gibco™), 1x penicillin streptomycin with glutamine (Gibco™), and 10 mM HEPES (Gibco™). Cells were passaged 3 times prior to addition of $\beta$-secretase sensors and inhibitors to ensure full recovery from thawing.

*Colocalization assays*

Dermal fibroblasts were passaged using 0.05 % trypsin-EDTA and subsequently counted using a CellDrop™ FL automated cell counter (Denovix®). 15,000 fibroblasts were seeded in 12 mm glass bottom dishes and allowed to recover overnight. Cells were then transduced with CellLight® BacMam vectors expressing either RFP-tagged Rab5, GFP-tagged Rab7, or RFP-tagged Lamp1 to enable visualization of early endosomes, late endosomes and lysosomes, respectively. Transductions were performed according to manufacturer's suggestions. In brief, volumes equating to 30 viral particles per cell were added directly to cell culture medium and cells were allowed to incubate for 16 h at 37 °C in 5 % $CO_2$. Cells were washed three times with 1 mL of PBS and pulsed with 1 $\mu$M of SecOn sensors for 30 min in 100 $\mu$l of labelling medium formulated with Hanks modified eagle medium (Gibco™), 1x NEAA (Gibco™), 10 mM HEPES (Gibco™), and 1x penicillin-streptomycin and glutamine (Gibco™). After the pulse period, cells were washed three times with PBS and then chased in chase medium comprising Phenol Red free, high glucose DMEM (25 mM glucose (Gibco™) supplemented with 10 % FBS (Gibco™), 1x NEAA (Gibco™), 1x penicillin-streptomycin with glutamine (Gibco™), 1 mM pyruvate (Gibco™) and 10 mM HEPES (Gibco™). For assessment of $\beta$-secretase sensor colocalization with early endosomes, images were acquired immediately after the 30 min pulse period and again after 3 h. For colocalization with late endosomes, images were acquired after 30 min pulse at 37 °C followed by a 3 h chase, while for colocalization with lysosomes, cells were chased for 6 h and images were acquired after. Fluorescent images were obtained with an Olympus® IX83 fluorescent microscope equipped with a 100x, 1.45 NA oil immersion objective and a Photometrics® Prime™-95B sCMOS camera.

*BACE1 activity inside cells*

BACE1 endosomal activity was assessed in dermal fibroblasts obtained from either healthy individuals (n=6), and Alzheimer's disease patients harbouring either a pathogenic PSEN1 mutation (*n*=3), an ApoE4 allele (*n*=3), or both a PSEN1 mutation and ApoE4 allele (*n*=3) (Table 3). 15,000 fibroblasts were seeded in 12 mm glass bottom dishes and allowed to recover overnight in proliferation medium. Proliferation medium was then removed from dishes and cells were washed three times with 1 mL of PBS. SecV and SecOff sensors (the HyLite-488 versions of SecV and SecOff, comprising $\beta$-SecOFF$^{V2}$ and $\beta$-sec$^{V2}$ respectively, were used for all cell-based experiments) were diluted to 1 $\mu$M in labelling medium (Serum free HBSS containing 1X NEAA and 10 mM HEPES) and 100 $\mu$L was added to dishes. Cells were pulsed for 1 h at 37 °C to permit optimal uptake. Next, labelling solutions were removed and cells were washed three times with 1 mL of PBS before chasing with 1.5 mL of chase medium for 3 h at 37 °C. Fluorescent images were obtained with an Olympus® IX83 fluorescent microscope equipped with a 100x, 1.45 Numerical Aperture (NA) oil immersion objective and a Photometrics® Prime™-95B sCMOS camera.

$$\text{Fold change of activity} = \frac{Mean\,\frac{G}{R}using\,\boldsymbol{SecV}}{Mean\,\frac{G}{R}using\,\boldsymbol{SecOff}}$$

*Table 3. Primary dermal fibroblasts used in β-secretase sensor performance*

| Coriell ID | Cohort | Age | Gender | Genetics |
|---|---|---|---|---|
| AG09908 | HI | 81 | Female | N/A |
| AG05844 | HI | 57 | Female | N/A |
| AG05192 | HI | 55 | Male | N/A |
| AG06294 | HI | 65 | Male | N/A |
| GM23974 | HI | 34 | Male | N/A |
| GM08400 | HI | 37 | Female | N/A |
| AG08110 | AD | 41 | Female | PS1 |
| AG04159 | AD | 52 | Female | PS1 |
| AG08555 | AD | 49 | Male | PS1 |
| AG07768 | At risk | 31 | Female | PS1 |
| AG09035 | At risk | 35 | Male | PS1 |
| AG08166 | At risk | 27 | Female | PS1 |
| AG06848 | AD | 56 | Female | PS1/E4 |
| AG08170 | AD | 56 | Male | PS1/E4 |
| AG06840 | AD | 56 | Male | PS1/E4 |
| AG05810 | AD | 79 | Female | E4 |
| AG04400 | AD | 61 | Female | E4 |
| AG06262 | AD | 66 | Male | E4 |

*Inhibitor treatment*

15,000 fibroblasts were seeded in 12 mm glass bottom dishes and allowed to recover overnight prior to addition of β-secretase or γ-secretase inhibitors. BSIV or DAPT was diluted to 25 μM or 50 μM, respectively, in proliferation medium and 1.5 mL was added to cells for 20 h. Proliferation medium was removed from dishes and cells were washed three times with 1 mL of PBS and 100 μL of labelling medium containing 1 μM of SecV and SecOff sensors was added to cells for 1 h at 37 °C. Labelling solutions were then removed, and cells were washed 3 times with 1 mL of PBS and chased for 3 h in inhibitors at 37 °C. G/R fluorescent images were captured using an Olympus IX83 fluorescent microscope equipped with a 100x, 1.45 NA oil immersion objective and a Photometrics® Prime™-95B sCMOS camera.

*Imaging of BACE1 activity*

All images were acquired using an Olympus IX83 motorized widefield microscope equipped with Apochromat 60x NA 1.42 and 100x NA 1.45 oil immersion objectives. Filter wheel, shutter and sCMOS camera were controlled using cellSens™ dimension version 3.2 imaging software. For *in vitro* BACE1 measurements, images were acquired in Green (G) and Red (R) channels. Fluorophores were excited with a Lumencore® SPECTRA X light source. Alexa Fluor 647 (R) channel images were acquired using 635/18 band pass excitation, 680/42 band pass emission filters and a FF409/493/573/652/759 dichroic mirror. EDANS or HyLite-488 (G) channel images were acquired using 378/52 band pass excitation and 432/36 band pass emission, or 474/27 band pass excitation and 515/30 band pass emission filters, respectively. For RFP or GFP colocalization experiments, images were acquired using 554/23 band pass excitation and 595/31 band pass emission, or 474/27 band pass excitation and 515/30 band pass emission filters, respectively. All images were acquired using a Photometrics® Prime™-95B sCMOS camera.

*Image analysis*

Image analysis for quantification of BACE1 activity in single endosomes was performed using custom python code. For all images, the Green channel (HyLite-488) was aligned to the Red (Alexa Fluor 647) channel by translating them to maximize the cross correlation between them. In the Red channel, the difference of the image with a Gaussian blurred version of itself was used to determine the boundaries of the Regions of Interest (ROIs). The ROIs were subsequently filtered based on area and intensity to remove artefacts. For each ROI, its immediate surrounding regions (which are not part of another ROI), were used to determine the background intensity to be subtracted from the intensity of the ROI for further calculations. Then, the mean fluorescence intensity in each endosome in FITC (G), and Cy5 647 (R) channels, and the background intensity corresponding to each respective cell and channel was subtracted. The ratio of intensities (G/R) were then computed for each endosome and exported as an excel file. Fold change of activity was calculated by dividing the mean G/R value from **SecV** by the mean G/R value of **SecOff** conditions for each cell line.

*Statistical methods*

Excel Software (Microsoft), Origin (OriginLab Corporation) were used to analyse and report the data. Statistical significance was calculated using two-tailed Student's t-test with Welch's correction for parametric data with unequal variance. Statistical differences between multiple conditions were performed by one- way ANOVA followed by Bonferroni's post-hoc tests. The comparison between different groups in experiments inside endolysosomal pathway was calculated by Estimation statistics[18] and significance was calculated by permutation *t-test.* Statistical significance was pre-fixed at $p< 0.05$, described as *$p < 0.05$; **$p< 0.01$; and ***$p< 0.001$. All experiments were performed at least in duplicates unless otherwise specified, and the mean between two replicates was used for statistical calculations.

**Results**

*Design of a DNA-based ratiometric β-Secretase (BACE1) activity reporter*

The developed sensor is an 'On' and 'Off' design, carrying two complementary strands (the sensing strand SecVn and the reference strand ES3). The design is divided into three independent sensors by varying the SecVn strand, as shown in **Figure 3.** To generate SecV, SecOff, and SecOn sensors, β-sec$^{V1}$/β-sec$^{V2}$, β-SecOFF$^{V1}$/β-SecOFF$^{V2}$ or β-SecON$^{V1}$/β-secON$^{V2}$ strands were employed as the SecVn strand respectively.

The SecV sensor (formed by annealing β-sec$^{V1}$and ES3 as described in Table 2 and Figure 11) is the BACE1 reporting sensor intended to measure real-time activity of the enzyme. SecV carries the β-sec$^{V1}$ strand which is a DNA-peptide conjugate carrying a BACE1 substrate peptide sequence that is accompanied by a FRET pair (Green circle, donor fluorophore, either EDANS or HyLite-488; orange circle, quencher, either DABCYL or QXL520). The ES3 strand is a complementary strand that carries a reference fluorophore (Red circle, Alexa Fluor 647N) for normalization purposes. In the presence of BACE1 the peptide substrate is cleaved which releases the quencher, resulting in an increase in fluorescence from the donor (unquenched state).

The Secoff sensor (formed by annealing β-SecOFF$^{V1}$ and ES3 as described in Table 2 and Figure 11) is a constitutively 'off' state sensor designed to measure basal signal coming from non-specific sources within the cells. This mimics the hypothetical state of the sensor when no BACE1 is present. It carries a dummy hexaethylene glycol (HEG) linker accompanied by the same FRET pair as BACE1 sensor SecV. The HEG strand is not cleaved by enzymes, thus donor intensity is quenched throughout the course of the reaction, providing a good reference sensor to measure enzyme 'off' (absent) state.

In *in vitro* experiments, a DNA duplex called SecOn (formed by annealing β-SecON$^{V1}$ and ES3 as described in Table 2 and Figure 11) was also employed to compare the maximum intensity observed from the SecV sensor. SecOn is a constitutively 'ON' sensor that lacks the quencher and indicates maximum signal obtained from the donor fluorophore. This mimics the hypothetical state where the reaction between the enzyme (BACE1) and its substrate reached saturation (i.e., 100 % of the quencher is cleaved from the substrate).

*In vitro BACE1 activity assay*

The three sensors were characterised in the presence and absence of BACE1 enzyme **(Figure 4A).** The intensity from the green (G, Donor) and red (R, Reference) fluorophore channels was measured and plotted as a ratio (G/R). As expected, the G/R ratio when using the SecOn sensor is higher than when using the Secoff sensor and both remain unaffected in the presence of BACE1. The G/R ratio from the SecV sensor remains similar to G/R ratios from the Secoff sensor in the absence of BACE1 which indicates complete quenching of the donor. In the presence of BACE1, the quencher is cleaved from the donor and fluorescent intensity in the green channel is increased leading to a high G/R

indicating the efficacy of the sensor in responding to the addition of BACE1. A >3.0-fold change in sensor response was observed when EDANS and DABCYL were used as a FRET pair and a >1.8-fold response when HyLite-488 and QXL520 were used as a FRET pair.

*In vitro SecV specificity*

**Figure 4B** describes the specificity of the SecV sensor to β-secretase. The G/R ratio in the absence of BACE1 remains low, and upon addition of BACE1 the sensor showed a 1.8-fold change in the signal indicating initiation of BACE1 mediated cleavage of SecV. When 300 nM of a potent BACE1 inhibitor, BSIV, was added to the reaction, the G/R ratio of the SecV is significantly reduced. Approximately 1.5-fold inhibition of the sensor signal is observed in the presence of BSIV indicating that SecV is highly specific to the activity of BACE1 enzyme. Importantly, the high G/R ratio of the SecV sensor is not inhibited in the presence of 800 nM of the γ-secretase inhibitor, DAPT, which suggests that inhibiting γ-secretase activity does not affect performance of the SecV sensor.

*In vitro SecV sensitivity*

**Figure 4C** demonstrates the sensitivity of SecV in the presence of BACE1. 3 μM of SecV was incubated with varying concentrations of BACE1 enzyme (0.4 ng to 640 ng). The SecV sensor showed detectable changes in signal from 8 ng and remains linear until 80 ng. This indicates that the SecV sensor can report BACE1 activity in solutions where the concentration is as low as 8 ng.

*Programmed trafficking of BACE1 sensor along endocytic pathway*

**Figure 5** shows the localization of BACE1 sensor at different time points of incubation within cells. Human dermal fibroblasts expressing Rab5 RFP were incubated with 1 μM DNA duplex (SecV) for 30 min (termed as pulse) at 37 °C and immediately imaged under a widefield microscope. **Figure 5A** describes colocalization of DNA sensors with early endosomal marker Rab5 indicating that upon 30 min incubation, SecV sensor accumulates within early endosomes. When cells were subjected to a further 180 min incubation at 37 °C (termed as chase) after initial labelling colocalization with Rab5 is no longer seen, indicating that SecV sensor is trafficked out of early endosomal compartments (Figure 6A). **Figure 5B** confirms localization of the SecOn sensor after 30 min pulse and 180 min of chase at 37 °C. SecOn showed colocalization with the late endosomal marker, Rab7 GFP, indicating that after 3 h incubation, SecOn reached late endosomes. **Figure 5C** shows localization of SecV sensor after a prolonged incubation for 360 min at 37 °C. When SecV sensor was subjected to 30 min pulse 37 °C and a further 6 h chase, they colocalized with lysosomal marker Lamp1 RFP. This indicates, that after 6 h of chase, SecV sensor reached lysosomal compartments.

**Figure 6A** shows quantification of colocalization of SecV with early endosomal marker Rab5 after 30 min pulse. Nearly 26 % DNA sensor is colocalized with early endosome. After a 180 min chase, the colocalization of SecV with Rab5 is significantly decreased. **Figure 6B** shows quantification of $SecOn_{647}$ with late endosomal marker Rab7 and lysosomal marker Lamp1. After 180 min chase, 83 % of DNA sensor is colocalized with late endosomes while after a 360 min of chase, nearly 78 % of DNA sensor is colocalized with lysosomes.

*Spatio-temporal measurement of BACE1 activity inside cells*

Upon labelling for 30 min (to mark early endosome) with SecV or labelling for 30 min at 37 °C followed by a 180 min and 300 min at 37 °C chase to mark late endosome and lysosome respectively, cells were imaged at 37 °C, and the ratio of G/R is quantified for reporting BACE1 activity within endo-lysosomal pathways.

**Figure 7A** shows a pseudocolour G/R map after different pulse and chase period. After imaging in Green (G) and Red (R) channels, the ratio of G/R in each pixel was pseudocoloured and represented. SecV sensor showed very low G/R at early endosomes (Blue pixels) which increased upon 180 min chase (at Late endosomes, Red pixels). Upon a further 180 min chase at 37 °C, the activity didn’t change significantly. This observation was quantified in **Figure 7B.** Mean G/R from >100 lysosomes from >10 cells were quantified. This data confirms that BACE1 activity is increased as endosomes matures to lysosomes.

*SecV specificity inside late endosomes*

**Figure 8A** describes specificity of the SecV sensor inside late endosomes. Cells were labelled for 60 min with SecV and SecOff sensors at 37 °C and chased for 180 min at 37 °C to label late endosomes in the presence of 25 μM BACE1 inhibitor (BSIV) or 50 μM γ-secretase inhibitor (DAPT). G/R pseudocolour map showed high G/R ratio reflecting increased BACE1 activity in the absence of BSIV, which is decreased in the presence of BSIV indicating the intracellular

signal is coming due to cleavage of SecV sensor by BACE1. Addition of DAPT did not change the G/R ratio that is remain similar to G/R values in DMSO control indicating the change in signal is coming specifically due to BACE1. This observation was further quantified in **Figure 8B.** The fold change of activity was calculated by normalizing mean SecV G/R ratio with the mean G/R ratio obtained from constitutively off sensor, SecOff from inhibitor treated or DMSO treated cells. The fold change of activity in untreated control was plotted and statistical comparison between treated and DMSO treated groups was performed using estimation statistics. The permutation *t*-testwas used to calculate significance between DMSO treated and inhibitor treated conditions.

*Monitoring BACE1 activity in cells from Alzheimer's disease patients*

Skin fibroblasts from 6 healthy individuals (HI) and 9 patients with AD were labelled with 1 μM SecV and SecOff for 60 min at 37 °C and chased for further 180 min 37 °C.

**Figure 9** shows pseudocolour G/R map after 60 min pulse and 180 min chase period at 37 °C. Constitutively off sensor SecOff showed very low G/R ratio which represents basal fluorescence coming from late endosomes in dermal fibroblasts. When cells were labelled with SecV sensor, the G/R ratio increases indicating activity of BACE1 in the healthy individuals (HI). The G/R ratio increases drastically in the familial form of the Alzheimer's disease (AD) carrying a mutation in *PSEN-1 (PS1)* gene. Sporadic form of AD did not show observable change in G/R ratio while, persons with a familial history of AD and with a 50 % risk of developing AD showed high G/R values from late endosomes.

**Figure 10A** quantifies the fold change of activity between HI and AD patients. G/R from individual lysosomes across >10 cells were quantified, and the mean ratio obtained from SecV was divided by mean G/R ratio calculated from SecOff and the resulting Fold change of activity was compared between HI and AD. The data showed significant increase in BACE1 activity in the AD patients.

**Figure 10B** quantifies BACE1 activity across different forms of AD. Familial AD patients showed significant increase in BACE1 activity compared to HI or AD patients carrying ApoE4 alleles. When comparing only Sporadic AD patients (Carrying at least one ApoE4 allele) with HI, no significant change in BACE1 activity was seen. Interestingly, it was observed that having a *PSEN-1* mutation along with ApoE4 allele significantly increases BACE1 activity which indicates that *PSEN-1* mutation alone has a significant effect on BACE1 activity in the late endosomes from the AD patients.

The present inventors hypothesized that, if having a *PSEN-1* mutation is enough to increase BACE1 activity, then individuals that are at risk of developing AD due to family history of AD and carrying a *PSEN-1* mutation should show increased activity. To prove this hypothesis, 3 individuals who had a familial history of AD and donated skin when they did not have the disease were chosen. **Figure 10C** describes fold change activity from these at risk (50 % risk according to repository) samples. When compared to HI, these 3 individuals showed significant increase BACE1 fold change of activity supporting our hypothesis that mutation in *PSEN-1* increases BACE1 activity. Notably, all of these 3 individuals later developed AD, suggesting that the BACE1 sensor (SecV) could be used to screen individuals to measure BACE1 activity.

**REFERENCES**

Bartzokis, G., Lu, P. H. & Mintz, J. Human brain myelination and amyloid beta deposition in Alzheimer's disease. Alzheimer's Dement. 3, 122-125 (2007).

Cai, H. et al. BACE1 is the major β-secretase for generation of Aβ peptides by neurons. Nat. Neurosci. 4, 233-234 (2001).

Chakraborty, K. et al. Tissue-specific targeting of DNA nanodevices in a multicellular living organism. eLife 10:e67830 (2021).

Deng Y, Wang Z, Wang R, Zhang X, Zhang S, Wu Y, Staufenbiel M, Cai F, Song W. Amyloid-β protein (Aβ) Glu11 is the major β-secretase site of β-site amyloid-β precursor protein-cleaving enzyme 1(BACE1) and shifting the cleavage site to Aβ Asp1 contributes to Alzheimer pathogenesis. Eur J Neurosci. (2013).

Dunn, B., Stein, P. & Cavazzoni, P. Approval of Aducanumab for Alzheimer Disease-The FDA's Perspective. JAMA Intern. Med. 181, 1276 (2021).

Götz, J., Chen, F., van Dorpe, J. & Nitsch, R. M. Formation of Neurofibrillary Tangles in P301L Tau Transgenic Mice Induced by Aβ42 Fibrils. Science (80). 293, 1491-1495 (2001).

He, Z. et al. Amyloid-β plaques enhance Alzheimer's brain tau-seeded pathologies by facilitating neuritic plaque tau aggregation. Nat. Med. 24, 29-38 (2018).

Hampel, H. et al. The Amyloid-β Pathway in Alzheimer's Disease. Mol. Psychiatry 26, 5481-5503 (2021).

Huse, J. T. et al. β-Secretase Processing in the Trans-Golgi Network Preferentially Generates Truncated Amyloid Species That Accumulate in Alzheimer's Disease Brain. J. Biol. Chem. 277, 16278-16284 (2002).

Modi, S et al. Two DNA nanomachines map pH changes along intersecting endocytic pathways inside the

same cell. Nature Nanotech 8, 459-467 (2013).

Sannerud, R. et al. ADP ribosylation factor 6 (ARF6) controls amyloid precursor protein (APP) processing by mediating the endosomal sorting of BACE1. Proc. Natl. Acad. Sci. 108, (2011).

Serrano-Pozo, A., Das, S. & Hyman, B. T. APOE and Alzheimer's disease: advances in genetics, pathophysiology, and therapeutic approaches. Lancet Neurol. 20, 68-80 (2021).

Zhang S, Wang Z, Cai F, Zhang M, Wu Y, Zhang J, Song W. BACE1 Cleavage Site Selection Critical for Amyloidogenesis and Alzheimer's Pathogenesis. J Neurosci. (2017).

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

All patents, patent applications and references mentioned throughout the specification of the present invention are herein incorporated in their entirety by reference.

The invention embraces all combinations of preferred and more preferred groups and suitable and more suitable groups and embodiments of groups recited above.

## Claims

1. A nucleic acid-based sensor comprising:

   (i) a sensing strand comprising a nucleic acid-peptide conjugate, a donor fluorophore and a quencher, wherein the peptide comprises a cleavable peptide sequence, and the donor fluorophore and quencher are conjugated to the nucleic acid-peptide conjugate such that cleavage of said peptide results in spatial separation of the donor fluorophore and quencher and an increase in fluorescence from the donor fluorophore; and
   (ii) a reference strand comprising a nucleic acid sequence complementary to the nucleic acid sequence of the sensing strand and a reference fluorophore.

2. The nucleic acid-based sensor as defined in claim 1, wherein the nucleic acid is DNA.

3. The nucleic acid-based sensor as defined in claim 1 or claim 2, wherein the quenching occurs by Fluorescence Resonance Energy Transfer (FRET).

4. The nucleic acid-based sensor as defined in any of claims 1 to 3, wherein the cleavable peptide sequence is a beta-site APP cleaving enzyme 1 (BACE1) sensitive peptide sequence, such as wherein the cleavable peptide sequence sensitivity is selective for BACE1, preferably wherein the cleavable peptide sequence is EVNLDAEF (SEQ ID NO: 1).

5. The nucleic acid-based sensor as defined in any of claims 1 to 4, wherein the donor fluorophore and reference fluorophore have different emission wavelengths.

6. The nucleic acid-based sensor as defined in any of claims 1 to 5, wherein the donor fluorophore and reference fluorophore are in a 1:1 ratio.

7. A method of determining the activity of an enzyme in a cell comprising:

   (i) providing the nucleic acid-based sensor as defined in any of claims 1 to 6 to the cell; and
   (ii) measuring the fluorescence intensity of the donor fluorophore.

8. The method as defined in claim 7, wherein the method further comprises measuring the fluorescence intensity of the reference fluorophore (R), and determining a normalised value for the fluorescence intensity of the donor fluorophore (D), wherein the normalised value is the ratio D/R.

9. The method as defined in claim 7 or claim 8, wherein in the activity is measured inside an organelle, such as wherein in the organelle is an acidic organelle, for example wherein in the organelle is a lysosome or an endosome.

10. The method as defined in any of claims 7 to 9, wherein in the cell is a neuron.

11. The method as defined in any of claims 7 to 10, wherein in the enzyme is BACE1.

**12.** A method of detecting, diagnosing, or monitoring a disease or disorder in a subject comprising:

(i) determining the activity of an enzyme in a cell obtained from said subject using the methods described in any of claims 7 to 11; and
(ii) comparing the activity measured in (i) to a control or reference level of activity to detect, diagnose or monitor the disease or disorder.

**13.** The method as defined in claim 12, wherein the disease or disorder is selected from: a neurodegenerative disease, PTSD, heart disease, epithelial cancers, diabetes, peroxisomal disorders.

**14.** The method as defined in claim 13, wherein the neurodegenerative disease is a neurodegenerative disease associated with increased generation of β-amyloid, such as wherein the disease or disorder is Alzheimer's disease.

**15.** A method of determining the effectiveness of an inhibitor of a target enzyme comprising:

(i) providing the enzyme inhibitor to the cell;
(ii) determining the activity of the target enzyme in a cell using the method as defined in any of claims 7 to 11; and
(iii) comparing the activity measured in said cell with a control, such that a reduction in activity compared to the control is indicative of the effectiveness of the inhibitor of the target enzyme.

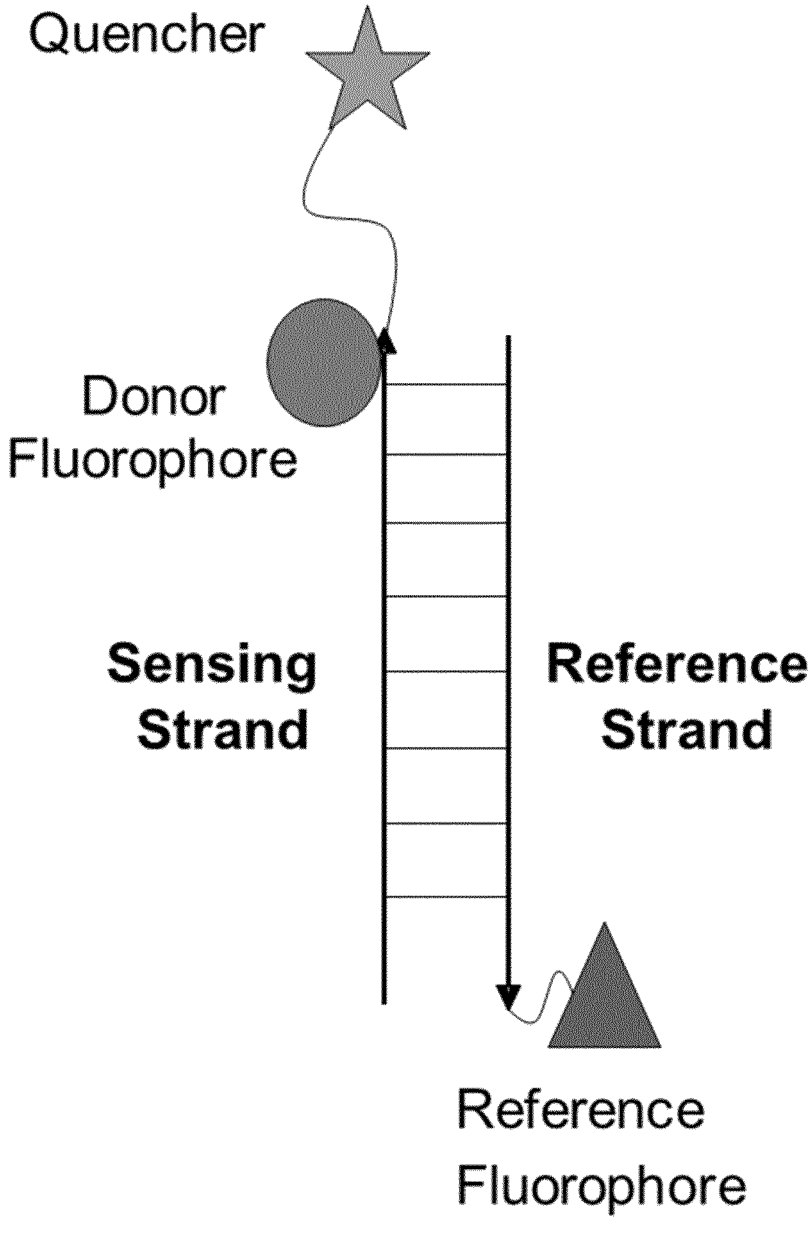
Quencher
Donor
Fluorophore
Sensing
Strand
Reference
Strand
Reference
Fluorophore

FIGURE 1

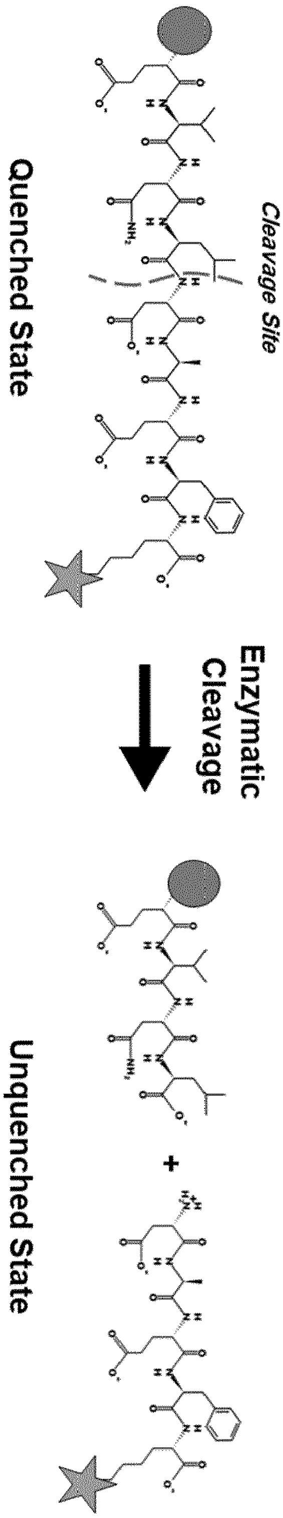
Quenched State
Cleavage Site
Enzymatic Cleavage
Unquenched State

FIGURE 2

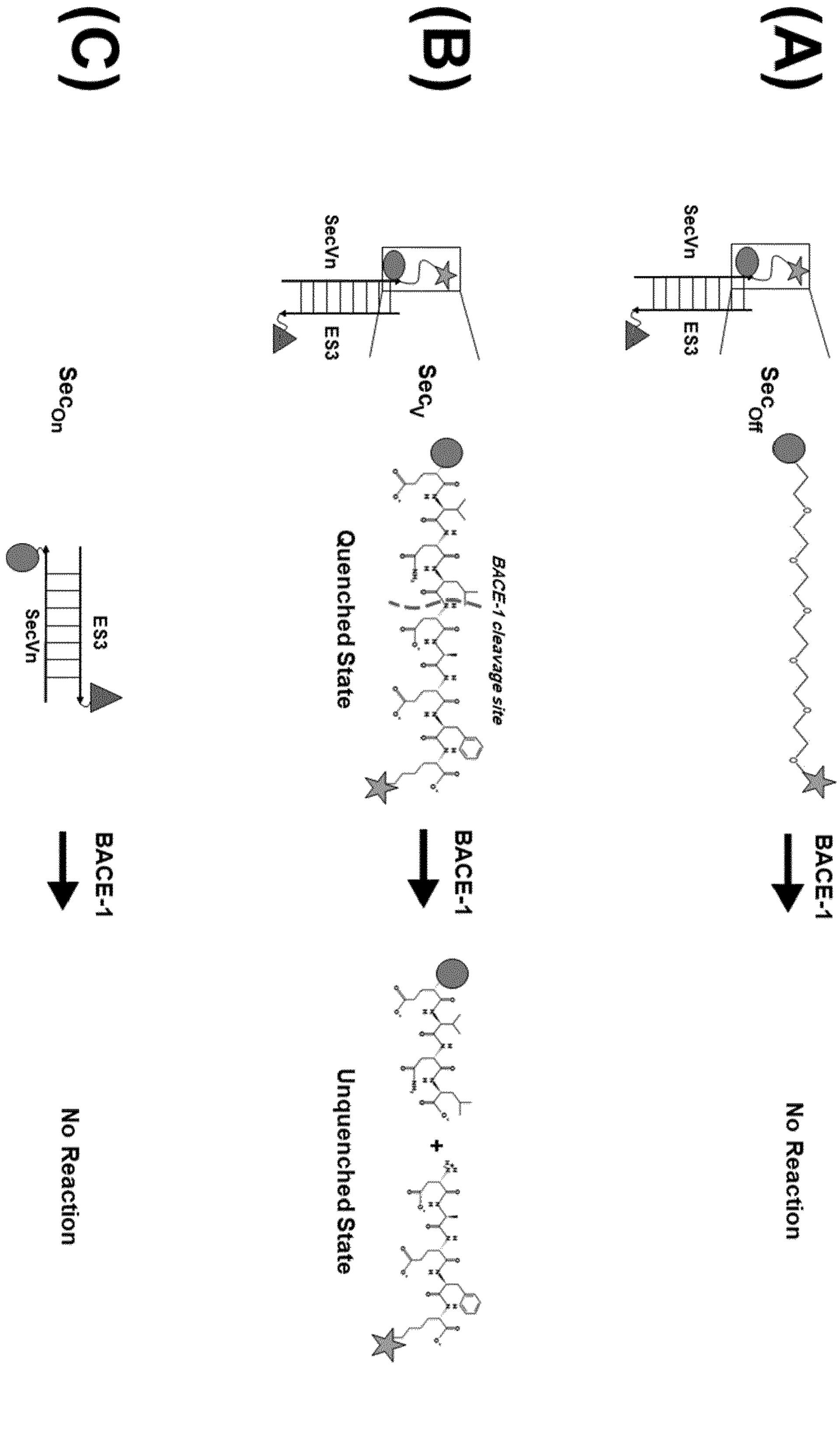
(A)
SecVn
ES3
Sec Off
BACE-1
No Reaction
(B)
SecVn
ES3
Sec V
BACE-1 cleavage site
Quenched State
BACE-1
+
Unquenched State
(C)
Sec On
ES3
SecVn
BACE-1
No Reaction

FIGURE 3

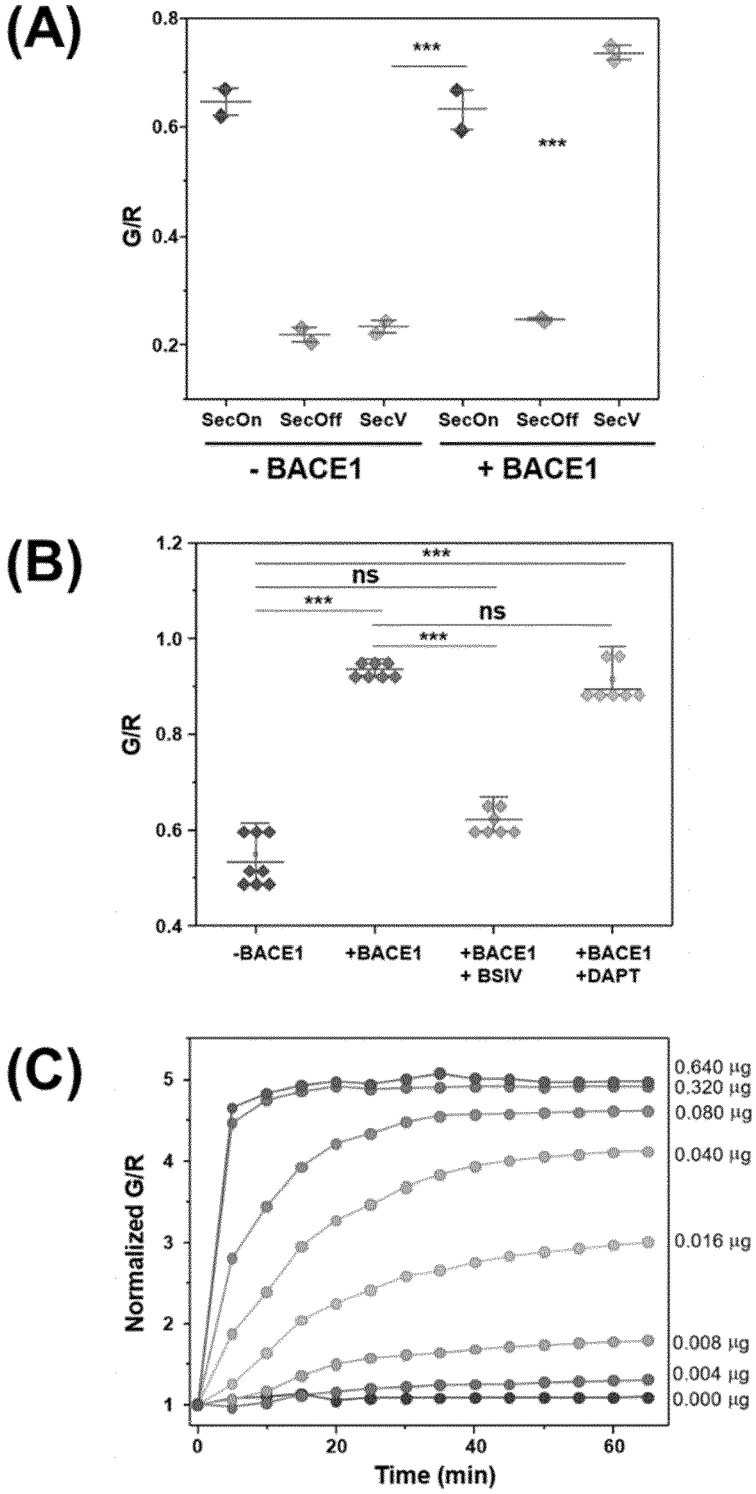
(A)
0.8
0.6
0.4
0.2
G/R
***
***
SecOn
SecOff
SecV
SecOn
SecOff
SecV
- BACE1
+ BACE1
(B)
1.2
1.0
0.8
0.6
0.4
G/R
***
ns
***
ns
***
-BACE1
+BACE1
+BACE1
+ BSIV
+BACE1
+DAPT
(C)
5
4
3
2
1
Normalized G/R
0
20
40
60
Time (min)
0.640 μg
0.320 μg
0.080 μg
0.040 μg
0.016 μg
0.008 μg
0.004 μg
0.000 μg

FIGURE 4

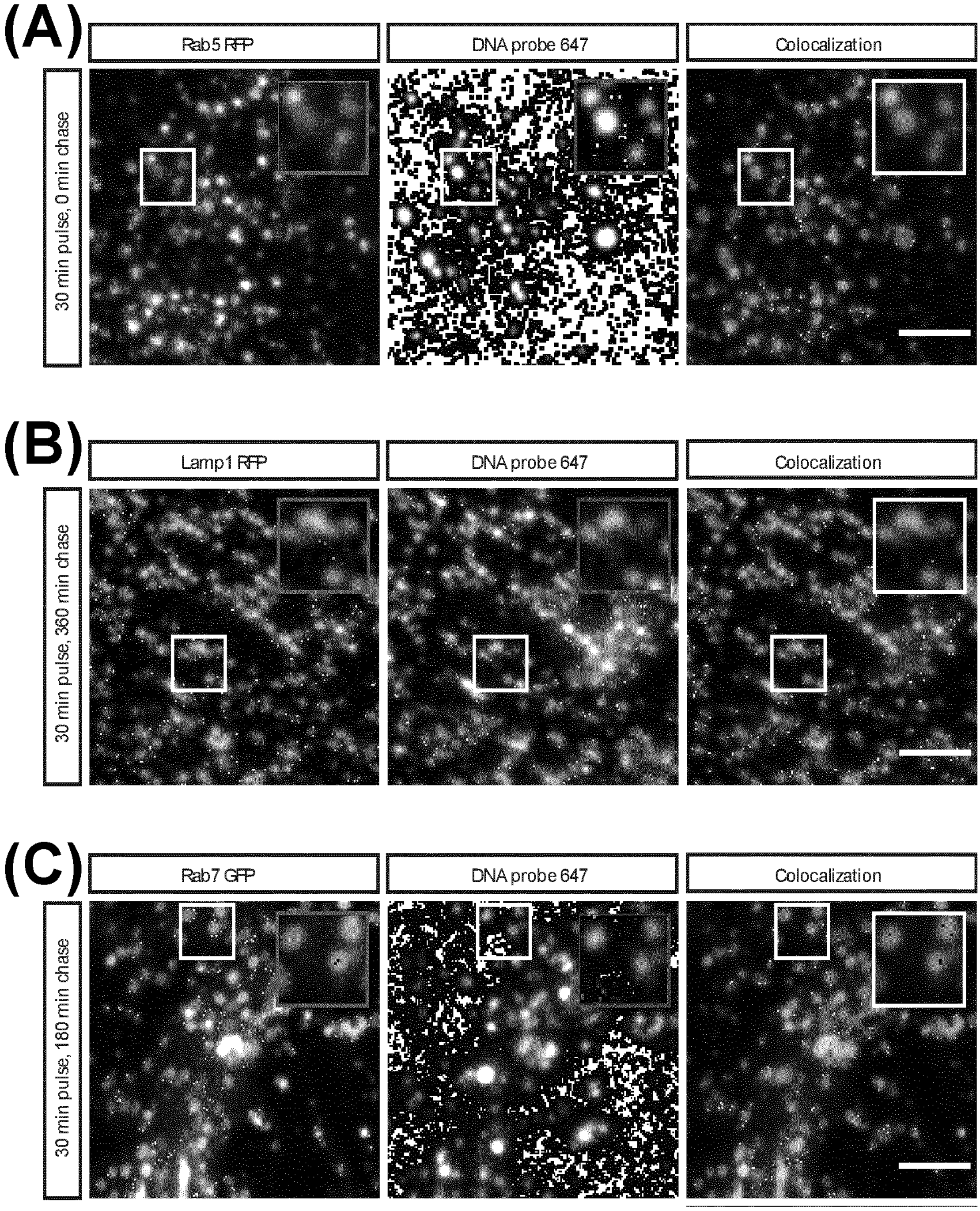
(A)
Rab5 RFP
DNA probe 647
Colocalization
30 min pulse, 0 min chase
(B)
Lamp1 RFP
DNA probe 647
Colocalization
30 min pulse, 360 min chase
(C)
Rab7 GFP
DNA probe 647
Colocalization
30 min pulse, 180 min chase

# FIGURE 5

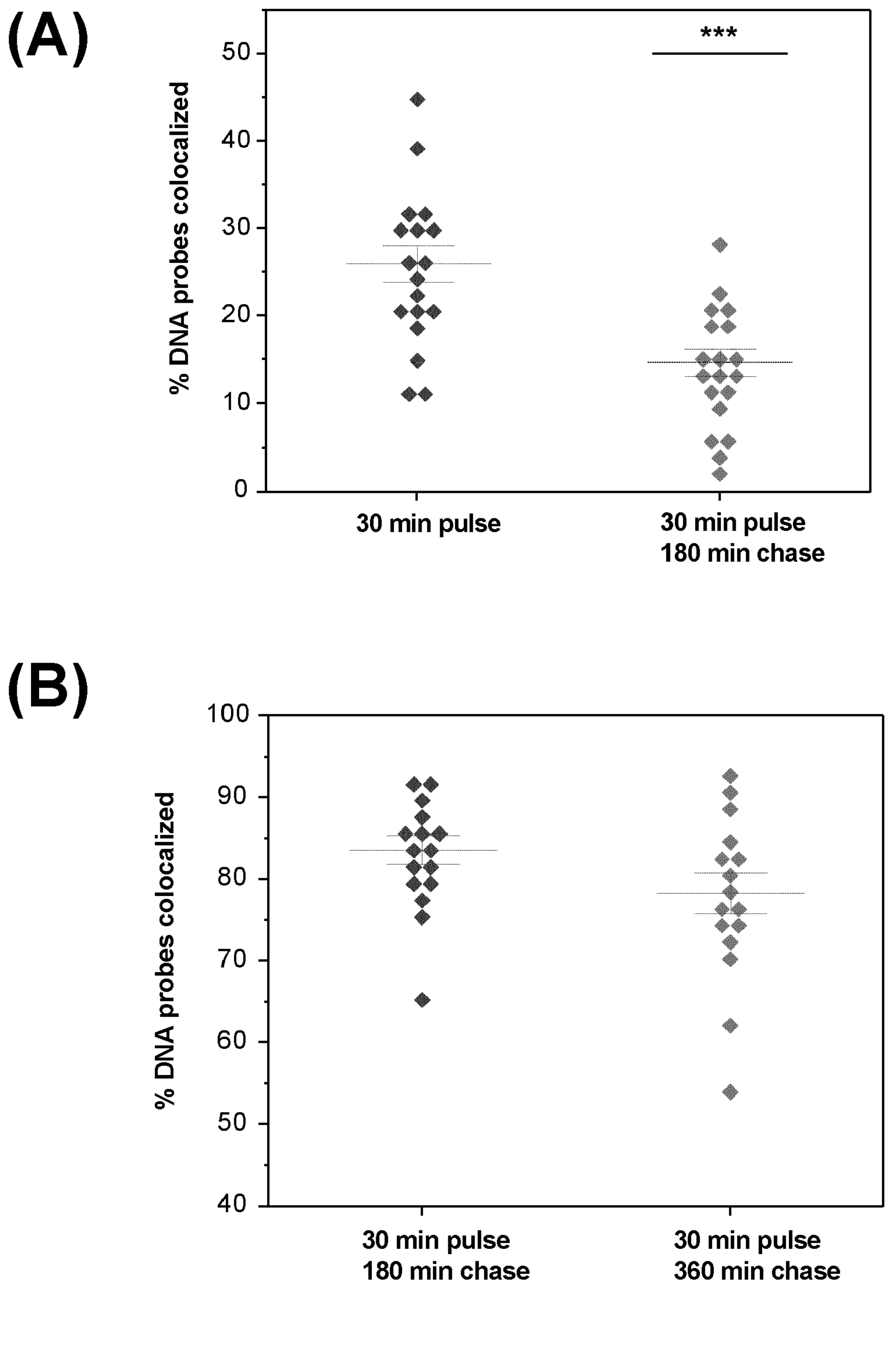
(A)
***
% DNA probes colocalized
0
10
20
30
40
50
30 min pulse
30 min pulse
180 min chase
(B)
% DNA probes colocalized
40
50
60
70
80
90
100
30 min pulse
180 min chase
30 min pulse
360 min chase

FIGURE 6

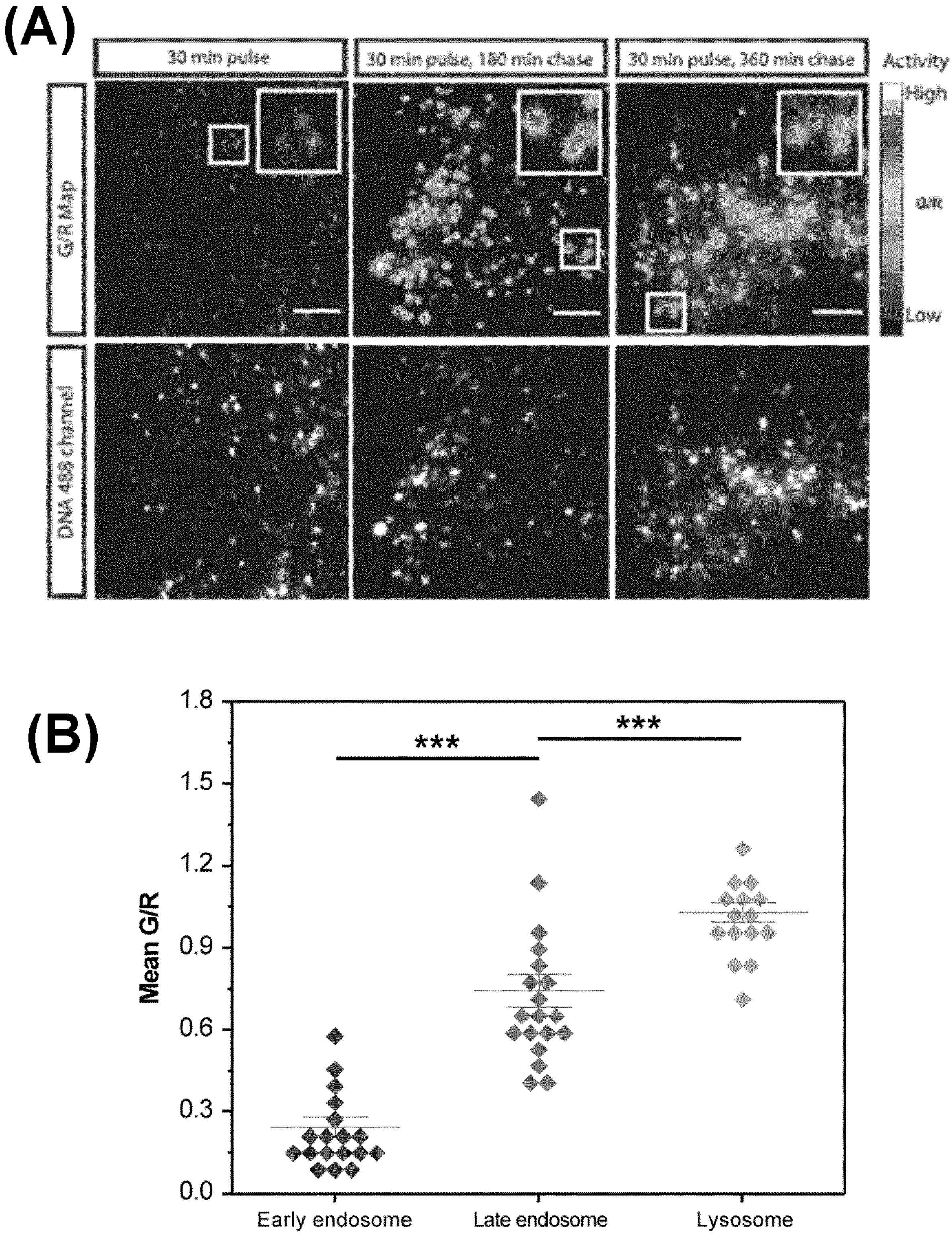
(A)
30 min pulse
30 min pulse, 180 min chase
30 min pulse, 360 min chase
Activity
High
G/R
Low
G/R Map
DNA 488 channel
(B)
1.8
1.5
1.2
0.9
0.6
0.3
0.0
Mean G/R
***
***
Early endosome
Late endosome
Lysosome

FIGURE 7

(A)

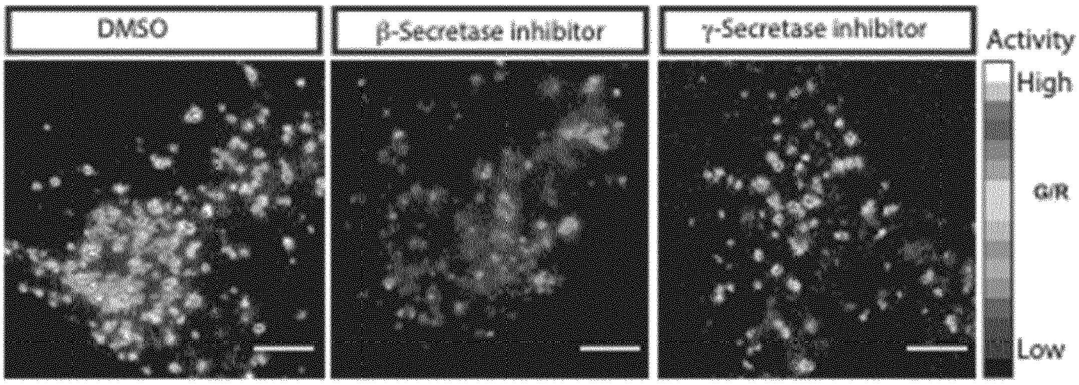
DMSO
β-Secretase inhibitor
γ-Secretase inhibitor
Activity
High
G/R
Low

(B)

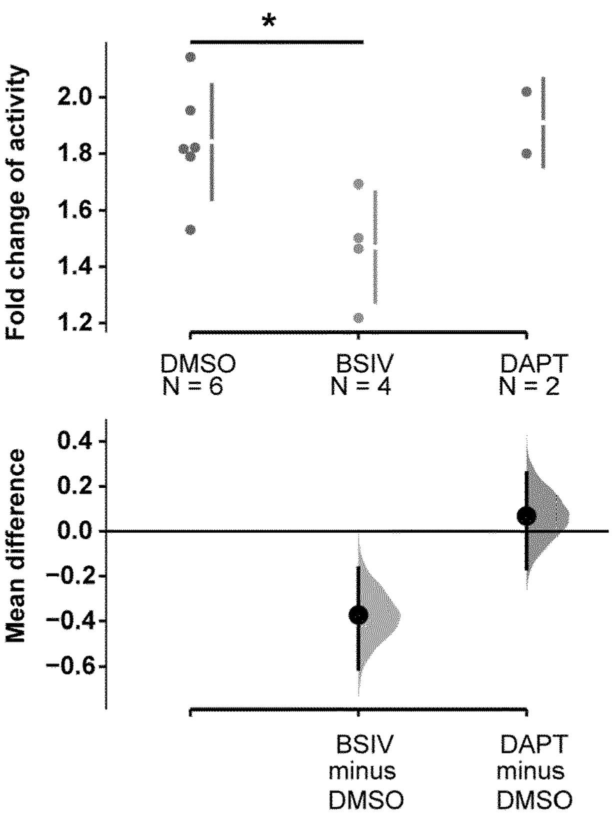
*
Fold change of activity
2.0
1.8
1.6
1.4
1.2
DMSO
N = 6
BSIV
N = 4
DAPT
N = 2
Mean difference
0.4
0.2
0.0
−0.2
−0.4
−0.6
BSIV
minus
DMSO
DAPT
minus
DMSO

FIGURE 8

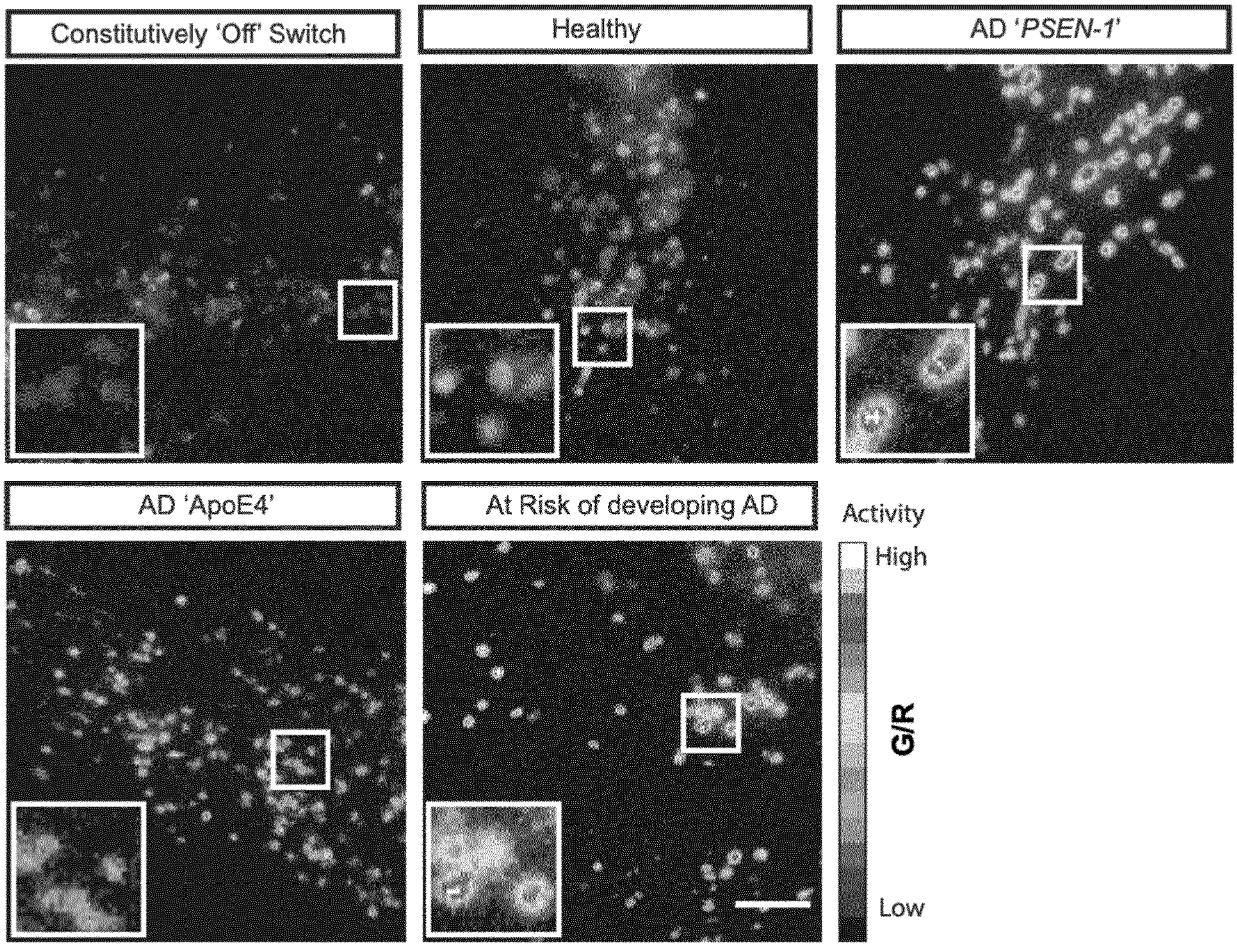
Constitutively 'Off' Switch
Healthy
AD 'PSEN-1'
AD 'ApoE4'
At Risk of developing AD
Activity
High
G/R
Low

# FIGURE 9

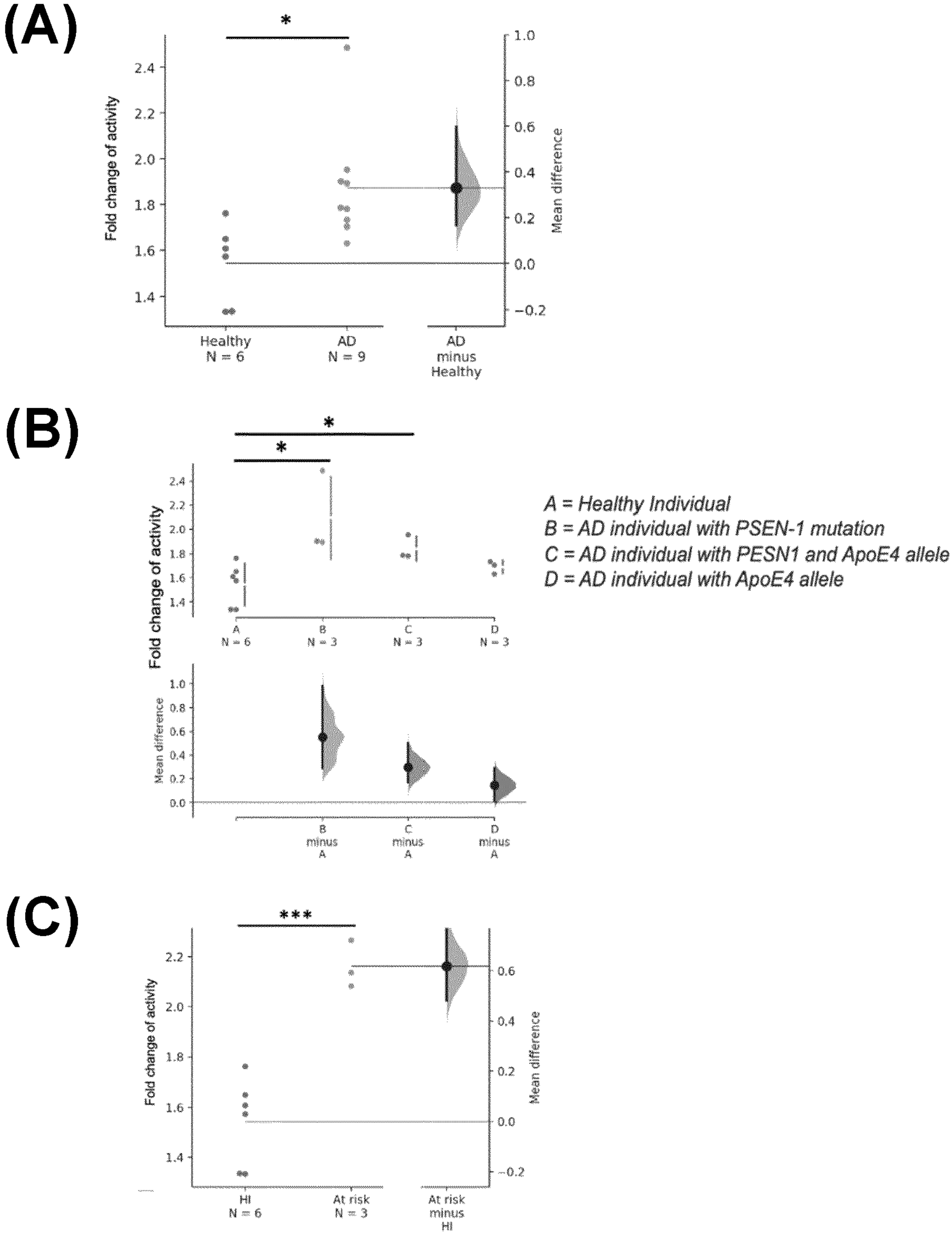
(A)
*
Fold change of activity
Mean difference
Healthy
N = 6
AD
N = 9
AD
minus
Healthy
(B)
*
*
Fold change of activity
A
N = 6
B
N = 3
C
N = 3
D
N = 3
A = Healthy Individual
B = AD individual with PSEN-1 mutation
C = AD individual with PESN1 and ApoE4 allele
D = AD individual with ApoE4 allele
Mean difference
B
minus
A
C
minus
A
D
minus
A
(C)
***
Fold change of activity
Mean difference
HI
N = 6
At risk
N = 3
At risk
minus
HI

FIGURE 10

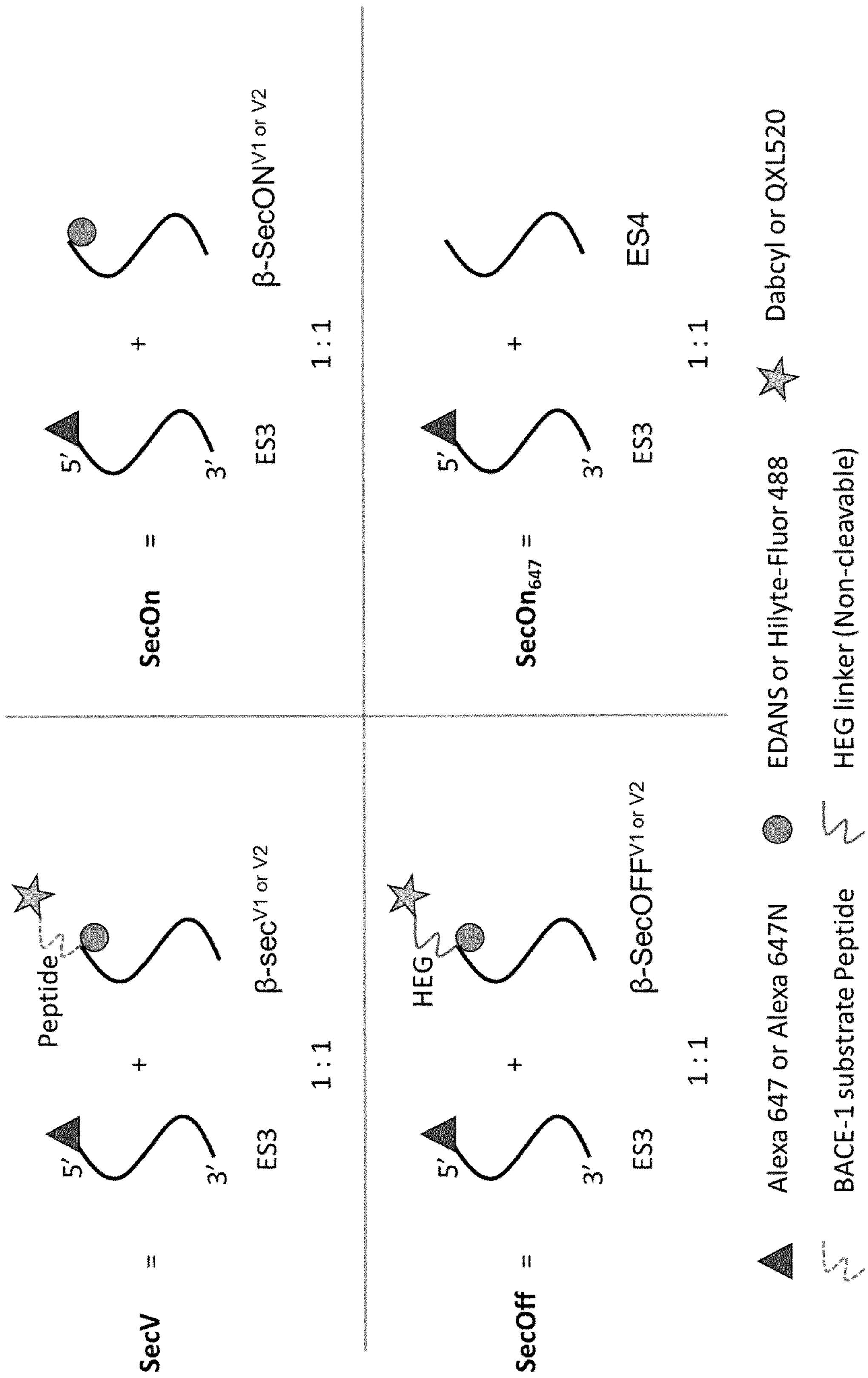
SecV =
5'
3'
ES3
+
Peptide
β-sec$^{V1\ or\ V2}$
1 : 1
SecOn =
5'
3'
ES3
+
β-SecON$^{V1\ or\ V2}$
1 : 1
SecOff =
5'
3'
ES3
+
HEG
β-SecOFF$^{V1\ or\ V2}$
1 : 1
SecOn$_{647}$ =
5'
3'
ES3
+
ES4
1 : 1
Alexa 647 or Alexa 647N
EDANS or Hilyte-Fluor 488
Dabcyl or QXL520
BACE-1 substrate Peptide
HEG linker (Non-cleavable)

FIGURE 11

European Patent Office

# EUROPEAN SEARCH REPORT

Application Number
EP 23 21 8578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 03/019138 A2 (QTL BIOSYSTEMS LLC [US]) 6 March 2003 (2003-03-06)<br>* claims 1-55; figures 1-10 *<br>----- | 1-15 | INV.<br>C12Q1/37<br>G01N33/573<br>G01N33/58<br>G01N33/68 |
| A | WO 2008/108873 A2 (UNIV SAINT LOUIS [US]; HEYDUK TOMASZ [US]; TIAN LING [US]) 12 September 2008 (2008-09-12)<br>* claims 1-40; figures 1-3, 38, 40 *<br>----- | 1-15 | |
| A | LIU LIN ET AL: "A graphene oxide-based fluorescent scheme for the determination of the activity of the [beta]-site amyloid precursor protein (BACE1) and its inhibitors",<br>MICROCHIMICA ACTA, SPRINGER VIENNA, VIENNA,<br>vol. 183, no. 1,<br>23 September 2015 (2015-09-23), pages 265-271, XP035887436,<br>ISSN: 0026-3672, DOI: 10.1007/S00604-015-1647-9<br>[retrieved on 2015-09-23]<br>Scheme 1;<br>* abstract *<br>----- | 1-15 | TECHNICAL FIELDS SEARCHED (IPC)<br>C12Q<br>G01N |
| A | WO 2018/191561 A1 (UNIV CHICAGO [US]) 18 October 2018 (2018-10-18)<br>* claims 1-52; figures 1, 14; example 3 *<br>-----<br>-/-- | 1-15 | |

1

The present search report has been drawn up for all claims

| Place of search | Date of completion of the search | Examiner |
|---|---|---|
| Munich | 8 May 2024 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 21 8578

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOI JIN-HA ET AL: "Metal-Enhanced Fluorescence by Bifunctional Au Nanoparticles for Highly Sensitive and Simple Detection of Proteolytic Enzyme", NANO LETTERS, vol. 20, no. 10, 18 August 2020 (2020-08-18), pages 7100-7107, XP055979741, US ISSN: 1530-6984, DOI: 10.1021/acs.nanolett.0c02343 * abstract; figure 1 * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 8 May 2024 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 23 21 8578

This annex lists the patent family members relating to the patent documents cited in the above-mentioned European search report. The members are as contained in the European Patent Office EDP file on The European Patent Office is in no way liable for these particulars which are merely given for the purpose of information.

08-05-2024

| Patent document cited in search report | Publication date | Patent family member(s) | Publication date |
|---|---|---|---|
| WO 03019138 A2 | 06-03-2003 | CA 2458405 A1 | 06-03-2003 |
| | | EP 1425581 A2 | 09-06-2004 |
| | | JP 2005501248 A | 13-01-2005 |
| | | KR 20040083411 A | 01-10-2004 |
| | | US 2003054413 A1 | 20-03-2003 |
| | | WO 03019138 A2 | 06-03-2003 |
| | | ZA 200402235 B | 29-08-2005 |
| WO 2008108873 A2 | 12-09-2008 | CA 2660129 A1 | 12-09-2008 |
| | | CN 101573452 A | 04-11-2009 |
| | | EP 2046998 A2 | 15-04-2009 |
| | | WO 2008108873 A2 | 12-09-2008 |
| WO 2018191561 A1 | 18-10-2018 | US 2021096129 A1 | 01-04-2021 |
| | | WO 2018191561 A1 | 18-10-2018 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BARTZOKIS, G. ; LU, P. H. ; MINTZ, J.** Human brain myelination and amyloid beta deposition in Alzheimer's disease. *Alzheimer's Dement,* 2007, vol. 3, 122-125
- **CAI, H. et al.** BACE1 is the major β-secretase for generation of Aβ peptides by neurons. *Nat. Neurosci.,* 2001, vol. 4, 233-234
- **CHAKRABORTY, K. et al.** Tissue-specific targeting of DNA nanodevices in a multicellular living organism. *eLife,* 2021, vol. 10, e67830
- **DENG Y ; WANG Z ; WANG R ; ZHANG X ; ZHANG S ; WU Y ; STAUFENBIEL M ; CAI F ; SONG W.** Amyloid-β protein (Aβ) Glu11 is the major β-secretase site of β-site amyloid-β precursor protein-cleaving enzyme 1(BACE1) and shifting the cleavage site to Aβ Asp1 contributes to Alzheimer pathogenesis. *Eur J Neurosci,* 2013
- **DUNN, B. ; STEIN, P. ; CAVAZZONI, P.** Approval of Aducanumab for Alzheimer Disease-The FDA's Perspective. *JAMA Intern. Med.,* 2021, vol. 181, 1276
- **GÖTZ, J. ; CHEN, F. ; VAN DORPE, J. ; NITSCH, R. M.** Formation of Neurofibrillary Tangles in P301L Tau Transgenic Mice Induced by Aβ42 Fibrils. *Science,* 2001, vol. 293 (80), 1491-1495
- **HE, Z. et al.** Amyloid-β plaques enhance Alzheimer's brain tau-seeded pathologies by facilitating neuritic plaque tau aggregation. *Nat. Med.,* 2018, vol. 24, 29-38
- **HAMPEL, H. et al.** The Amyloid-β Pathway in Alzheimer's Disease. *Mol. Psychiatry,* 2021, vol. 26, 5481-5503
- **HUSE, J. T. et al.** β-Secretase Processing in the Trans-Golgi Network Preferentially Generates Truncated Amyloid Species That Accumulate in Alzheimer's Disease Brain. *J. Biol. Chem.,* 2002, vol. 277, 16278-16284
- **MODI, S et al.** Two DNA nanomachines map pH changes along intersecting endocytic pathways inside the same cell. *Nature Nanotech,* 2013, vol. 8, 459-467
- **SANNERUD, R. et al.** ADP ribosylation factor 6 (ARF6) controls amyloid precursor protein (APP) processing by mediating the endosomal sorting of BACE1. *Proc. Natl. Acad. Sci.,* 2011, vol. 108
- **SERRANO-POZO, A. ; DAS, S. ; HYMAN, B. T.** APOE and Alzheimer's disease: advances in genetics, pathophysiology, and therapeutic approaches. *Lancet Neurol,* 2021, vol. 20, 68-80
- **ZHANG S ; WANG Z ; CAI F ; ZHANG M ; WU Y ; ZHANG J.** Song W. BACE1 Cleavage Site Selection Critical for Amyloidogenesis and Alzheimer's Pathogenesis. *J Neurosci,* 2017